# EUROPEAN PATENT APPLICATION

(11) **EP 4 458 323 A1**
(43) Date of publication of application: **06.11.2024**
(21) Application number: 21969773.7
(22) Date of filing: 31.12.2021
(51) Int. Cl.: A61F 2/24

(54) **ATRIAL SHUNT APPARATUS AND IMPLANTATION METHOD THEREFOR, ATRIAL IMPLANTATION DEVICE, MEDICAL SYSTEM, CARDIAC ACTIVITY INFORMATION COLLECTION METHOD, AND MEDICAL DEVICE**

(71) Applicant: United Innomed (Shanghai) Limited, Shanghai 201303 (CN)
(72) Inventor: WANG, Li, Shanghai 201203 (CN)
(74) Representative: Murgitroyd & Company
(86) International application number: PCT/CN2021/143873
(87) International publication number: WO 2023/123428

(57) **Abstract**

The present disclosure provides an atrial shunt apparatus (A-1) and a mounting method thereof, an atrial implanting device (B-10, C-10, C-20, C-30), a medical system (B-100), a method for collecting activity information of a heart (B-S100), and a medical device. This atrial shunt apparatus (A-1) is used for shunting blood from a left atrium (LA) to a right atrium (RA) through a hole (H) at an atrial septum (AS) of a patient. The atrial shunt apparatus (A-1) includes a shunt part (A-11), the shunt part (A-11) defines a shunt pathway (A-111), and the shunt pathway (A-111) has a first opening (A-111a) and a second opening (A-111b). The shunt pathway (A-111) is configured to communicate with the left atrium (LA) and the right atrium (RA) through the first opening (A-111a) and the second opening (A-111b) respectively, the first opening (A-111a) is configured to be closer to a head of the patient than the second opening (A-1 1 1b), and/or the first opening (A-111a) is configured to be closer to a front of a body of the patient relative to the second opening (A-111b). This atrial shunt apparatus (A-1) provided by the present disclosure effectively prevents or reduces blood and/or a suspended substance in the blood from entering into the left atrium (LA) from the right atrium (RA), and there is no problem of pathway blockage.

## Description

### TECHNICAL FIELD

The present disclosure relates to the technical field of medical instruments, and in particular, to an atrial shunt apparatus and a mounting method thereof, an atrial implanting device, a medical system, a method for collecting activity information of a heart and a medical device.

### BACKGROUND

Heart failure, abbreviated as HF, which refers to that due to the dysfunction of a systolic function and/or a diastolic function of a heart, the venous return blood volume cannot be fully discharged out of the heart, resulting in blood stasis of a venous system and insufficient blood perfusion of an arterial system, thereby causing a cardiac circulatory disturbance syndrome. Clinical studies have found that most of the heart failure begins with left heart failure. Increased pressure in a left atrium, or even continuous high pressure, is an important inducement of the left heart failure. For a patient with heart failure who has sustained high pressure in a left atrium at rest, or whose left atrium pressure is significantly increased and right atrium pressure is relatively low during exercise, blood may be shunted from a left atrium to a right atrium by creating a hole at an atrial septum and mounting an atrial shunt apparatus, and thus the left atrium pressure is effectively reduced, and the heart failure symptom of the patient is alleviated.

Heart disease is a class of circulatory disease. Among internal medicine diseases, the heart disease is most common, which can significantly affect the activity capability of a patient, greatly reduce the quality of life of the patient, increase the requirements for hospitalization, and even seriously threaten the life safety of the patient. With the development of medical technology, treatment methods for various heart disease are constantly emerging. For example, for a patient with heart failure who has sustained high pressure in a left atrium, a hole may be made at a atrial septum of the patient and an atrial shunt apparatus may be mounted at the hole, to make blood flow between a left atrium and a right atrium (for the purpose of making the blood flow from the left atrium to the right atrium), and thus the left atrium pressure is reduced and the symptom of the heart failure is alleviated. For another example, for a patient with an atrial septal defect, a plugging apparatus may be disposed at an opening of an atrial septum, thereby plugging the flow of blood between a left atrium and a right atrium, and restoring a pathway of normal blood circulation.

Among internal medicine diseases, the heart disease is most common, which can significantly affect labor capacity of a patient, greatly reduce life quality of the patient, and seriously threaten life safety of the patient. Some heart diseases will cause the heart beat slowly and even cause cardiac arrest. For problems of slow heart beat and cardiac arrest, the current most common treatment is to implant a cardiac pacemaking apparatus. In a conventional cardiac pacemaking apparatus, two electrodes are typically implanted in the atria and ventricle of a patient, to achieve dual-chamber pace-making of the heart. Over the years, leadless pace-making technologies have been developed, but currently, the technology in clinical use is limited to the leadless pace-making of the ventricle. However, since the ventricular pace-making is non-physiological pace-making, long-term use of the ventricular pace-making (particularly pace-making of the right ventricular apex) will result in pacemaker syndrome, thereby leading to deterioration of cardiac status, and then endangering health of a patient. This limits wide clinical application of the leadless pace-making technologies.

### SUMMARY

In a first aspect, an atrial shunt apparatus is provided. This atrial shunt apparatus is used for shunting blood from a left atrium to a right atrium through a hole at an atrial septum of a patient. The atrial shunt apparatus includes a shunt part, the shunt part defines a shunt pathway, the shunt pathway has a first opening and a second opening. The shunt pathway is configured to communicate with the left atrium and the right atrium through the first opening and the second opening respectively, the first opening is configured to be closer to a head of the patient relative to the second opening, and/or the first opening is configured to be closer to a front of a body of the patient relative to the second opening.

Combined with the first aspect, in some embodiments, the first opening is configured to be closer to the head and the front of the body of the patient relative to the second opening.

Combined with the first aspect, in some embodiments, the first opening is configured to be closer to the head and the front of the body of the patient relative to the hole, and the second opening is configured to be closer to a foot and a back of the body of the patient relative to the hole.

Combined with the first aspect, in some embodiments, the first opening is configured so that an orthographic projection of the first opening on a plane where the atrial septum is located is located in a first quadrant of a hypothetical coordinate system, and the second opening is configured so that an orthographic projection of the second opening on the plane where the atrial septum is located is located in a third quadrant of the coordinate system. The coordinate system is a plane rectangular coordinate system, the coordinate system takes the plane where the atrial septum is located as a reference plane, takes the hole as an origin, takes an intersection line of a horizontal plane passing through the origin when the patient is in an upright posture and the reference surface as an X axis, takes a direction from a back to the front of the body of the patient as a positive direction of the X axis, takes a straight line passing through the origin and perpendicular to the X axis, on the reference surface, as a Y axis, and takes a direction from a foot to the head of the body of the patient as a positive direction of the Y axis.

Combined with the first aspect, in some embodiments, the first opening is configured so that an included angle, between the x axis and a connecting line between an orthographic projection of the first opening on the reference surface and the origin, ranges from 30 degrees to 60 degrees, and the second opening is configured so that an included angle, between the x axis and a connecting line between an orthographic projection of the second opening on the reference surface and the origin, ranges from 30 degrees to 60 degrees.

Combined with the first aspect, in some embodiments, the first opening is configured so that the included angle, between the x axis and the connecting line between the orthographic projection of the first opening on the reference surface and the origin, is 45 degrees, and the second opening is configured so that the included angle, between the x axis and the connecting line between the orthographic projection of the second opening on the reference surface and the origin, is 45 degrees.

Combined with the first aspect, in some embodiments, the first opening is configured so that, an orthographic projection, of the first opening on a plane where the atrial septum is located, at least partially coincides with the hole, and the second opening is configured to be closer to a foot and a back of the body of the patient relative to the hole.

Combined with the first aspect, in some embodiments, the first opening is configured so that, an orthographic projection of the first opening on a plane where the atrial septum is located, substantially coincides with an origin of a hypothetical coordinate system, and the second opening is configured so that, an orthographic projection of the second opening on the plane where the atrial septum is located is located, is located in a third quadrant of the coordinate system. The coordinate system is a plane rectangular coordinate system, the coordinate system takes the plane where the atrial septum is located as a reference surface, takes the hole as an origin, takes an intersection line of a horizontal plane passing through the origin when the patient is in an upright posture and the reference surface as an X axis, takes a direction from a back to the front of the body of the patient as a positive direction of the X axis, takes a straight line passing through the origin and perpendicular to the X axis, on the reference surface, as a Y axis, and takes a direction from a foot to the head of the body of the patient as a positive direction of the Y axis.

Combined with the first aspect, in some embodiments, the second opening is configured so that, an included angle, between the x axis and a connecting line between an orthographic projection of the second opening on the reference surface and the origin, ranges from 30 degrees to 60 degrees.

Combined with the first aspect, in some embodiments, the second opening is configured so that, the included angle, between the x axis and the connecting line between the orthographic projection of the second opening on the reference surface and the origin, is 45 degrees.

Combined with the first aspect, in some embodiments, the shunt part is tubular, the shunt part includes a first portion pipe body and a second portion pipe body, the first portion pipe body is configured to be arranged in the left atrium, the second portion pipe body is configured to be arranged in the right atrium, the first opening is arranged at an end face or a peripheral wall of the first portion pipe body, and the second opening is arranged on an end face or a peripheral wall of the second portion pipe body.

Combined with the first aspect, in some embodiments, the shunt part is tubular, the shunt part includes a first portion pipe body and a second portion pipe body, the first portion pipe body is configured to be arranged in the left atrium, the second portion pipe body is configured to be arranged in the right atrium, the first opening and the second opening are respectively arranged on the first portion pipe body and the second portion pipe body, and volume of the first portion pipe body is less than volume of the second portion pipe body.

Combined with the first aspect, in some embodiments, the shunt part is tubular, the shunt part includes a first portion pipe body and a second portion pipe body, the first portion pipe body is configured to be arranged in the left atrium, the second portion pipe body is configured to be arranged in the right atrium, the first opening and the second opening are respectively arranged on the first portion pipe body and the second portion pipe body, and the first portion pipe body is shorter than the second portion pipe body.

Combined with the first aspect, in some embodiments, the first opening is configured to be higher than the second opening in a direction of gravity when the patient is in an upright and a supine posture, and a height difference between the first opening and the second opening in the direction of the gravity direction ranges from 1.5 cm to 2 cm.

Combined with the first aspect, in some embodiments, an inner diameter of the shunt pathway ranges from 8 mm to 15 mm.

Combined with the first aspect, in some embodiments, the inner wall of the shunt pathway is hydrophobic and/or resistant to formation of neoplasma.

Combined with the first aspect, in some embodiments, the atrial shunt apparatus further includes a frame part, the frame part has a mesh frame, and the frame part includes: a first frame part, which is configured to be arranged in the left atrium; a second frame part, which is configured to be arranged in the right atrium; and a connecting part, which connects the first frame part and the second frame part, and is configured to pass through the hole. The shunt part is tubular, and the shunt part includes: a first portion pipe body, the first opening is arranged on the first portion pipe body, and the first portion pipe body is configured to be surrounded by the first frame part; and a second portion pipe body, the second opening is arranged on the second portion pipe body, and the second portion pipe body is configured to be surrounded by the second frame part.

Combined with the first aspect, in some embodiments, the atrial shunt apparatus further includes a frame part, the frame part includes: a first frame part, which is configured to be arranged in the left atrium; a second frame part, which is configured to be arranged in the right atrium; and a connecting part, which connects the first frame part and the second frame part, and is configured to pass through the hole. The shunt part is tubular, and the shunt part includes: a first portion pipe body, the first opening is arranged on the first portion pipe body, the first portion pipe body is configured so that a portion of the first portion pipe body is arrange in the first frame part, and the other portion of the first portion pipe body extends out of the first frame part, to make the first opening be exposed out of the frame part; and a second portion pipe body, the second opening is arranged on the second portion pipe body, the second portion pipe body is configured so that a portion of the second portion pipe body is arrange in the second frame part, and the other portion of the second portion pipe body extends out of the second frame part, to make the second opening be exposed out of the frame part.

Combined with the first aspect, in some embodiments, the atrial shunt apparatus further includes a frame part, and the frame part includes: a first frame part, which is configured to be arranged in the left atrium; a second frame part, which is configured to be arranged in the right atrium; and a connecting part, which connects the first frame part and the second frame part, and is configured to pass through the hole. The shunt part is tubular, and the shunt part includes: a first portion pipe body, which is configured to extend from an inner of the first frame part to a surface of the first frame part, and form the first opening on the surface of the first frame part; and a second portion pipe body, which is configured to extend from an inner of the second frame part to a surface of the second frame part, and form the second opening on the surface of the second frame part.

Combined with the first aspect, in some embodiments, the first frame part is configured so that, an orthographic projection, of the first frame part on a plane where the atrial septum is located, covers an orthographic projection, of the first portion pipe body and the second portion pipe body on the plane where the atrial septum is located, and the second frame part is configured so that, an orthographic projection, of the second frame part on the plane where the atrial septum is located, covers the orthographic projection of the first portion pipe body and the second portion pipe body on the plane where the atrial septum is located.

Combined with the first aspect, in some embodiments, the first portion pipe body is configured so that, an orthographic projection, of the first portion pipe body on a plane where the atrial septum is located, is at least partially located outside an orthographic projection of the second frame part on the plane where the atrial septum is located, and an orthographic projection, of the second portion pipe body on the plane where the atrial septum is located, is at least partially located outside an orthographic projection of the first frame part on the plane where the atrial septum is located.

Combined with the first aspect, in some embodiments, the first frame part is less than the second frame part.

Combined with the first aspect, in some embodiments, the first frame part and/or the second frame part is dome-shaped.

In a second aspect, an atrial shunt apparatus is provided. This atrial shunt apparatus is used for shunting blood to a right atrium from a left atrium through a hole at an atrial septum of a patient. The atrial shunt apparatus includes a shunt part, the shunt part defines a shunt pathway, and the shunt pathway has a first opening and a second opening. The shunt pathway is configured to communicate with the left atrium and the right atrium through the first opening and the second opening respectively, the first opening is configured to be higher than the second opening in a direction of gravity when the patient is in an upright posture, and/or the first opening is configured to be higher than the second opening in a direction of gravity when the patient is in a supine posture.

In a third aspect, an atrial shunt apparatus is provided. This atrial shunt apparatus includes a shunt part, the shunt part defines a shunt pathway, and the shunt pathway has a first opening and a second opening. The shunt part is tubular, and the shunt part includes: a first portion pipe body, the first opening is arranged on the first portion pipe body; a second portion pipe body, the second opening is arranged on the second portion pipe body; and a third portion pipe body, an end of the third portion pipe body communicates with the first portion pipe body, and the other end of the third portion pipe body communicates with the second portion pipe body, the first portion pipe body is substantially parallel to the second portion pipe body, and the first portion pipe body and the second portion pipe body form an included angle with the third portion pipe body respectively; and the first portion pipe body and the second portion pipe body respectively extend from the third portion pipe body along opposite directions.

In a fourth aspect, an atrial shunt apparatus is provided. This atrial shunt apparatus includes a shunt part, the shunt part defines a shunt pathway, and the shunt pathway has a first opening and a second opening. The shunt part is tubular, and the shunt part includes: a first portion pipe body, the first opening is arranged on an end face of the first portion pipe body; and a second portion pipe body, the second opening is arranged on the second portion pipe body, an included angle is formed between the second portion pipe body and the first portion pipe body, and the first portion pipe body is shorter than the second portion pipe body.

In a fifth aspect, a method for mounting an atrial shunt apparatus is provided. This method for mounting the atrial shunt apparatus includes: providing the atrial shunt apparatus, where the atrial shunt apparatus is used for shunting blood from a left atrium to a right atrium through a hole at an atrial septum of a patient, the atrial shunt apparatus includes a shunt part, the shunt part defines a shunt pathway, and the shunt pathway has a first opening and a second opening; and mounting the atrial shunt apparatus to atria of the patient, the shunt pathway is configured to communicate with the left atrium and the right atrium through the first opening and the second opening respectively, the first opening is configured to be closer to a head of the patient relative to the second opening, and/or the first opening is configured to be closer to a front of a body of the patient relative to the second opening.

In a sixth aspect, a method for mounting an atrial shunt apparatus is provided. This method for mounting the atrial shunt apparatus includes: providing the atrial shunt apparatus, where the atrial shunt apparatus is used for shunting blood from a left atrium to a right atrium through a hole at an atrial septum of a patient, the atrial shunt apparatus comprises a shunt part, the shunt part defines a shunt pathway, and the shunt pathway has a first opening and a second opening; and mounting the atrial shunt apparatus in atria of the patient, where the shunt pathway is configured to communicate with the left atrium and the right atrium through the first opening and the second opening respectively, the first opening is configured to be higher than the second opening in a direction of gravity when the patient is at an upright and a supine posture, and/or the first opening is configured to be higher than the second opening in a direction of gravity when the patient is in a supine posture.

In a seventh aspect, an atrial implanting device is provided. This atrial implanting device includes: a treatment apparatus, which is configured to be arranged at an opening of an atrial septum of a patient, and is used for allowing blood to flow between a left atrium and a right atrium of the patient, or for preventing blood from flowing between a left atrium and a right atrium of the patient; and a collection apparatus, which is configured to be arranged on the treatment apparatus, and is used for collecting activity information of a heart of the patient.

Combined with the seventh aspect, in some embodiments, the collection apparatus includes a hemodynamic information collection unit, and the activity information includes hemodynamic information collected by the hemodynamic information collection unit.

Combined with the seventh aspect, in some embodiments, the hemodynamic information includes pressure information, the hemodynamic information collection unit includes a vibrating diaphragm for sensing the pressure information, and the vibrating diaphragm is configured to be directly exposed to blood in the left atrium or be directly exposed to blood in the right atrium.

Combined with the seventh aspect, in some embodiments, the vibrating diaphragm is configured to be parallel to the atrial septum.

Combined with the seventh aspect, in some embodiments, the hemodynamic information collection unit has a first end and a second end which are opposite to each other, the vibrating diaphragm is arranged at the first end, and a direction from the first end to the second end of the hemodynamic information collection unit is configured to be the same as or opposite to a blood flow direction.

Combined with the seventh aspect, in some embodiments, the hemodynamic information collection unit has a first end and a second end which are opposite to each other, the vibrating diaphragm is arranged at the first end, a direction from the first end to the second end is perpendicular to the atrial septum, and the first end is farther from the atrial septum relative to the second end.

Combined with the seventh aspect, in some embodiments, the treatment apparatus includes a shunt member, the shunt member is provided with a first opening, a second opening and a shunt pathway which extends from the first opening to the second opening, the first opening and the second opening are configured to be respectively located in the left atrium and the right atrium, to allow the blood to flow between the left atrium and the right atrium, and the hemodynamic information collection unit is configured to be located at an edge of the first opening or the second opening.

Combined with the seventh aspect, in some embodiments, the treatment apparatus includes a shunt member, and the shunt member defines a shunt pathway for communicating with the left atrium and the right atrium, to allow the blood to flow between the left atrium and the right atrium, and the hemodynamic information collection unit is configured to be located in the shunt pathway.

Combined with the seventh aspect, in some embodiments, the collection apparatus includes two hemodynamic information collection units, the two hemodynamic information collection units are configured to be located in the shunt pathway, and in the shunt pathway, in a direction from the left atrium to the right atrium, a vibrating diaphragm of one hemodynamic information collection unit is located at an upstream side of the one hemodynamic information collection unit, and a vibrating diaphragm of the other one hemodynamic information collection unit is located at a downstream side of the other one hemodynamic information collection unit.

Combined with the seventh aspect, in some embodiments, a housing of the two hemodynamic information collection units is integrally formed.

Combined with the seventh aspect, in some embodiments, the collection apparatus further includes an intracardiac electrocardiogram collection unit, and the activity information further includes intracardiac electrocardiogram information collected by the intracardiac electrocardiogram collection unit.

Combined with the seventh aspect, in some embodiments, the intracardiac electrocardiogram collection unit includes a first electrode, and a part or all of a housing of the hemodynamic information collection unit constitutes the first electrode.

Combined with the seventh aspect, in some embodiments, the intracardiac electrocardiogram collection unit further includes a second electrode, the housing of the hemodynamic information collection unit includes a first portion housing and a second portion housing which are electrically separated from each other, the first portion housing constitutes the first electrode, and the second portion housing constitutes the second electrode.

Combined with the seventh aspect, in some embodiments, the treatment apparatus includes a shunt member, the shunt member defines a shunt pathway for connecting the left atrium and the right atrium, to allow blood to flow between the left atrium and the right atrium, the intracardiac electrocardiogram collection unit includes a first electrode, and a part or all of the shunt member constitutes the first electrode.

Combined with the seventh aspect, in some embodiments, the intracardiac electrocardiogram collection unit further includes a second electrode, the second electrode is configured to be in direct contact with the atrial septum, the atrial implanting device further includes a spacer, and the spacer is configured to electrically separate the second electrode from the shunt member.

Combined with the seventh aspect, in some embodiments, the treatment apparatus includes a shunt member and a frame, the shunt member defines a shunt pathway for communicating with the left atrium and the right atrium, to allow blood to flow between the left atrium and the right atrium, the frame is used for supporting the shunt member, the intracardiac electrocardiogram collection unit includes a first electrode, and a part or all of the frame constitutes the first electrode.

Combined with the seventh aspect, in some embodiments, the intracardiac electrocardiogram collection unit further includes a second electrode, the frame includes a first frame part and a second frame part which are electrically separated from each other, the first frame part and the second frame part are configured to be respectively located in the left atrium and the right atrium, the first frame part constitutes the first electrode, and the second frame part constitutes the second electrode.

Combined with the seventh aspect, in some embodiments, the collection apparatus further includes a calibration unit, and the calibration unit is used for synchronously writing time calibration information into the hemodynamic information and the intracardiac electrocardiogram information respectively.

In an eighth aspect, a medical system is provided. This medical system includes: any one of the atrial implanting devices described in above the seventh aspect; and an external device which is configured to be located outside a patient, the external device including a communication unit, and the communication unit is used for receiving the activity information from the atrial implanting device.

Combine with the eighth aspect, in some embodiments, the external device further includes a body surface electrocardiogram collection unit, and the body surface electrocardiogram collection unit is used for collecting electrocardiogram information of a body surface the patient.

In a ninth aspect, a medical system is provided. This medical system includes: any one of the atrial implanting devices described in above the seventh aspect; and an external device which is configured to be located outside a patient, the external device includes a body surface electrocardiogram collection unit and a calibration unit. The body surface electrocardiogram collection unit is used for collecting body surface electrocardiogram information of the patient; and the calibration unit is used for generating and sending time calibration information to the hemodynamic information collection unit and the body surface electrocardiogram collection unit, to make the hemodynamic information collection unit and the body surface electrocardiogram collection unit synchronously write the time calibration information into the hemodynamic information and the body surface electrocardiogram information.

In a tenth aspect, a medical system is provided. This medical system includes: any one of the atrial implanting devices described in the above seventh aspect; and an external device, which is configured to be located outside a patient. The external device includes a body surface electrocardiogram collection unit and a calibration unit. The body surface electrocardiogram collection unit is used for collecting body surface electrocardiogram information of the patient; and the calibration unit is used for generating and sending time calibration information to the hemodynamic information collection unit, the intracardiac electrocardiogram collection unit and the body surface electrocardiogram collection unit, to make the hemodynamic information collection unit, the intracardiac electrocardiogram collection unit and the body surface electrocardiogram collection unit synchronously write the time calibration information into the hemodynamic information, the intracardiac electrocardiogram information and the body surface electrocardiogram information.

In an eleventh aspect, a method for collecting activity information of a heart is provided. The method for collecting the activity information of the heart includes: acquiring hemodynamic information in atria of a patient; acquiring electrocardiogram information of the patient; and analyzing the hemodynamic information and the electrocardiogram information, to obtain an analysis result.

In a twelfth aspect, a medical device is provided. This medical device includes: a memory; and a processor, which is coupled to the memory. The processor is configured to execute the method for collecting the activity information of the heart described in above the eleventh aspect based on an instruction stored in the memory.

In a thirteenth aspect, an atrial implanting device is provided, this atrial implanting device includes: a pacemaking apparatus, where the pacemaking apparatus includes a pulse generation module, a first electrode and a housing, the pulse generation module is accommodated in the housing, the first electrode is configured to be in contact with an atrial septum, and the pulse generation module is configured to deliver a pacemaking pulse to the atrial septum through the first electrode; and an anchoring apparatus, which is configured to anchor the housing to the atrial septum.

Combined with the thirteenth aspect, in some embodiments, a part or all of the housing constitutes a second electrode of the pacemaking apparatus.

Combined with the thirteenth aspect, in some embodiments, the anchoring apparatus includes a shunt apparatus, the shunt apparatus is configured to communicate with the left atrium and the right atrium through an opening at the atrial septum, and the pacemaking apparatus is arranged on the shunt apparatus.

Combined with the thirteenth aspect, in some embodiments, the shunt apparatus includes a shunt member, the shunt member forms a shunt pathway for communicating with a left atrium and a right atrium, the pacemaking apparatus further includes a second electrode, and all or a part of the shunt member constitutes the second electrode.

Combined with the thirteenth aspect, in some embodiments, the shunt apparatus includes a shunt member and a mesh frame, the shunt member forms a shunt pathway for communicating with a left atrium and a right atrium, and the mesh frame covers the housing and the shunt member.

Combined with the thirteenth aspect, in some embodiments, the pacemaking apparatus further includes a second electrode, and a part or all of the mesh frame constitutes the second electrode.

Combined with the thirteenth aspect, in some embodiments, the atrial implanting device further includes a spacer, and the spacer is configured between the first electrode and the mesh frame, to electrically separate the first electrode from the mesh frame.

Combined with the thirteenth aspect, in some embodiments, the shunt apparatus includes a shunt member, the shunt member is formed with a first opening, a second opening and a shunt pathway which extends between the first opening and the second opening, the first opening is configured in a left atrium of a patient, the second opening is configured in a right atrium of the patient, and the first opening is configured to be closer to a head of the patient and a front of the body of the patient relative to the second opening.

Combined with the thirteenth aspect, in some embodiments, the anchoring apparatus includes a plugging apparatus, the plugging apparatus is configured at an opening of the atrial septum and plugs the opening, and the housing is mounted on the plugging apparatus.

Combined with the thirteenth aspect, in some embodiments, the atrial implanting device further includes a collection apparatus, the collection apparatus is used for collecting activity information of a heart of the patient, and the anchoring apparatus is configured to anchor the collection apparatus to the atrial septum.

Combined with the thirteenth aspect, in some embodiments, the housing is configured in the right atrium.

### BRIEF DESCRIPTION OF THE DRAWINGS

In order to more clearly illustrate the technical solutions of embodiments of the present disclosure, the drawings required to be used in the embodiments are briefly described below. It should be understood that, the following drawings merely show some embodiments of the present disclosure, and therefore should not be intended to limit the scope, and for a person with ordinary skill in the art, other related drawings may be obtained according to these drawings without creative efforts.

It should be understood that, the same or similar reference numerals are used in the drawings to refer to the same or similar members.

It should be understood that, the drawings are merely illustrative, and dimensions and proportions of the members in the drawings are not necessarily precise.
FIG. 1 is a schematic structural diagram of an atrial shunt apparatus configured in atria according to some embodiments of the present disclosure.
FIG. 2 is a schematic structural diagram of another view direction of FIG. 1 in which the atrial shunt apparatus is configured in the atria.
FIG. 3 is a schematic structural diagram of another view direction of FIG. 1 in which the atrial shunt apparatus is configured in the atria.
FIG. 4 is a schematic structural diagram of a shunt part according to some embodiments of the present disclosure.
FIG. 5 is a schematic structural diagram of an atrial shunt apparatus configured in atria according to some other embodiments of the present disclosure.
FIG. 6 is a schematic structural diagram of an atrial shunt apparatus configured in atria according to some other embodiments of the present disclosure.
FIG. 7 is a schematic structural diagram of an atrial shunt apparatus configured in atria according to some other embodiments of the present disclosure.
FIG. 8 is a schematic structural diagram of another view direction of FIG. 7 in which the atrial shunt apparatus is configured in the atria.
FIG. 9 is a schematic structural diagram of another view direction of FIG. 7 in which the atrial shunt apparatus is configured in the atria.
FIG. 10 is a schematic structural diagram of a shunt part according to some other embodiments of the present disclosure.
FIG. 11 is a schematic structural diagram of an atrial shunt apparatus configured in atria according to some other embodiments of the present disclosure.
FIG. 12 is a schematic structural diagram of a frame part according to some embodiments of the present disclosure.
FIG. 13 is a schematic structural diagram of an atrial shunt apparatus configured in atria according to some other embodiments of the present disclosure.
FIG. 14 is a schematic structural diagram of a frame part according to some other embodiments of the present disclosure.
FIG. 15 is a schematic structural diagram of an atrial shunt apparatus configured in atria according to some other embodiments of the present disclosure.
FIG. 16 is a schematic structural diagram of a frame part according to some other embodiments of the present disclosure.
FIG. 17 is a schematic structural diagram of an atrial shunt apparatus configured in atria according to some other embodiments of the present disclosure.
FIG. 18 is a schematic structural diagram of an atrial shunt apparatus configured in atria according to some other embodiments of the present disclosure.
FIG. 19 is a schematic structural diagram of an atrial shunt apparatus configured in atria according to some other embodiments of the present disclosure.
FIG. 20 is a schematic flow diagram of a method for mounting an atrial shunt apparatus according to some embodiments of the present disclosure.
FIG. 21 is a schematic structural diagram of an atrial implanting device according to an embodiment of the present disclosure.
FIG. 22 is a schematic structural diagram of an atrial implanting device according to another embodiment of the present disclosure.
FIG. 23 is a schematic structural diagram of an atrial implanting device according to another embodiment of the present disclosure.
FIG. 24 is a schematic structural diagram of an atrial implanting device according to another embodiment of the present disclosure.
FIG. 25 is a schematic structural diagram of an atrial implanting device according to another embodiment of the present disclosure.
FIG. 26 is a schematic structural diagram of an atrial implanting device according to another embodiment of the present disclosure.
FIG. 27 is a schematic structural diagram of an atrial implanting device according to another embodiment of the present disclosure.
FIG. 28 is a schematic structural diagram of an atrial implanting device according to another embodiment of the present disclosure.
FIG. 29 is a schematic structural diagram of an atrial implanting device according to another embodiment of the present disclosure.
FIG. 30 is a schematic structural diagram of an atrial implanting device according to another embodiment of the present disclosure.
FIG. 31 is a schematic structural diagram of an atrial implanting device according to another embodiment of the present disclosure.
FIG. 32 is a schematic structural diagram of a shunt member configured in atria of a patient according to an embodiment of the present disclosure.
FIG. 33 is a schematic structural diagram of another view direction of FIG. 32 in which the shunt member is configured in the atria of the patient.
FIG. 34 is a schematic structural diagram of another view direction of FIG. 32 in which the shunt member is configured in the atria of the patient.
FIG. 35 is a schematic structural diagram of an atrial implanting device according to another embodiment of the present disclosure.
FIG. 36 is a schematic structural diagram of an atrial implanting device according to another embodiment of the present disclosure.
FIG. 37 is a schematic structural diagram of a collection apparatus according to an embodiment of the present disclosure.
FIG. 38 is a schematic structural diagram of a medical system according to an embodiment of the present disclosure.
FIG. 39 is a schematic flowchart of a method for collecting information of a heart according to an embodiment of the present disclosure.
FIG. 40 is a schematic structural diagram of a medical device according to an embodiment of the present disclosure.
FIG. 41 is a schematic diagram of an atrial implanting device according to an embodiment of the present disclosure.
FIG. 42 is a schematic diagram of a pacemaking apparatus according to an embodiment of the present disclosure.
FIG. 43 is a schematic diagram of an atrial implanting device according to another embodiment of the present disclosure.
FIG. 44 is a schematic diagram of a shunt member according to an embodiment of the present disclosure.
FIG. 45 is another schematic diagram of the shunt member in FIG. 44.
FIG. 46 is another schematic diagram of the shunt member in FIG. 44.
FIG. 47 is a schematic diagram of an atrial implanting device according to another embodiment of the present disclosure.

### DETAILED DESCRIPTIONS OF THE EMBODIMENTS

### Exemplary atrial shunt apparatus and mounting method thereof

In an ideal situation, the atrial shunt apparatus should provide merely one flow direction of blood, that is, the blood can merely flow from a left atrium to a right atrium, but cannot flow from the right atrium to the left atrium. If the blood flows from the right atrium to the left atrium, a health problem will be caused. For example, if thrombus or other particle in venous blood is not filtered by a pulmonary capillary and enters into the left atrium directly through an opening of an atrial septum, and then enters into the arterial blood circulation (including cerebral circulation) through the left atrium, it will lead to that a patient has problems such as cerebral stroke, subacute stroke, or blockage of small artery blood vessels of an organ and the like.

In order to prevent the blood from flowing from the right atrium into the left atrium, an implementation is to provide a valve in a blood flow pathway of an atrial shunt apparatus, this kind of valve has a function similar to a one-way valve, which merely allows the blood to flow from the left atrium to the right atrium. However, clinical studies have shown that, for this type of atrial shunt apparatus, most patients will have blockage or partial blockage in the pathway of the atrial shunt apparatus at the valve after it is used for some time, and thus the atrial shunt apparatus is caused to fail or its expected effect is reduced.

To solve the above-mentioned problem, an embodiment of the application provides an atrial shunt apparatus. The atrial shunt apparatus according to the embodiment of the present disclosure will be illustrated with reference to the drawings below.

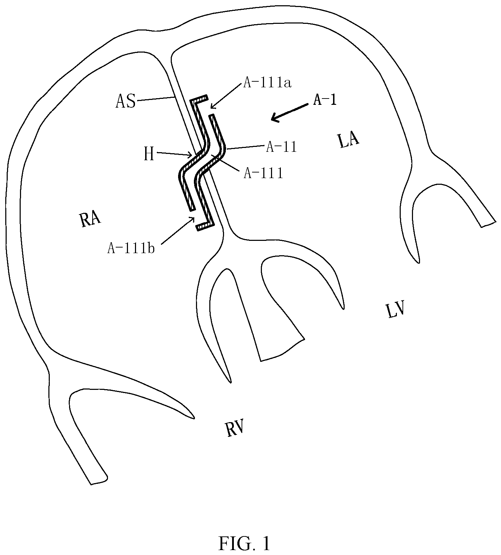

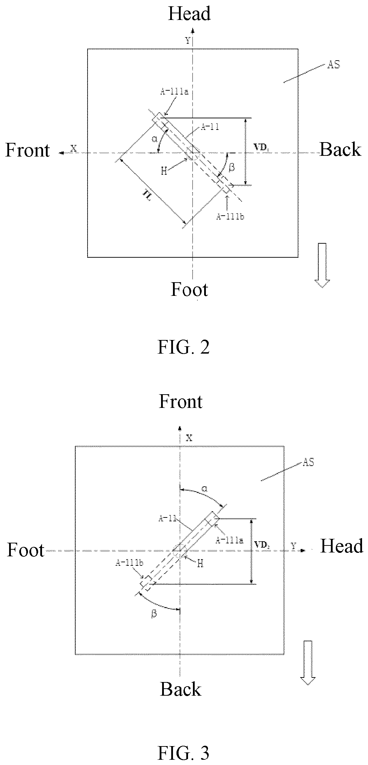

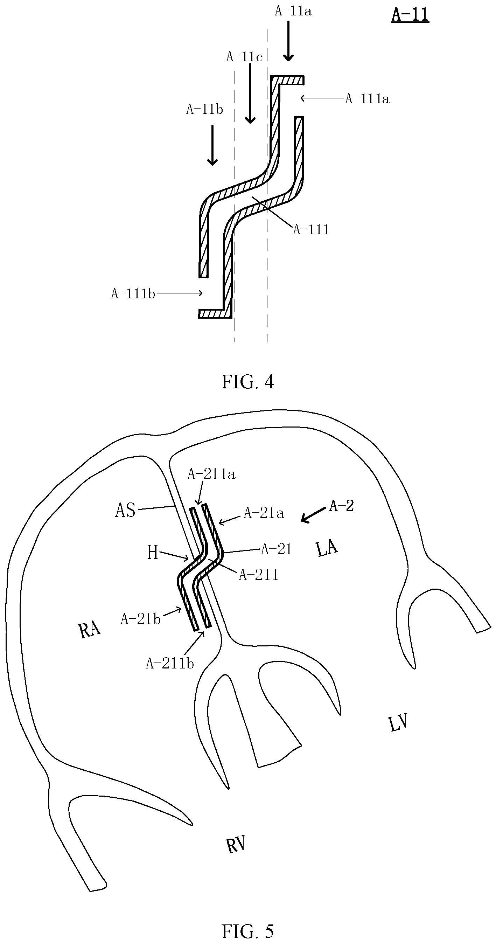

FIG. 1 is a schematic structural diagram of an atrial shunt apparatus A-1 configured in atria according to some embodiments of the present disclosure. FIG. 2 and FIG. 3 are schematic structural diagrams of other view directions of the FIG. 1 in which the atrial shunt apparatus is configured in the atrium. FIG. 4 is a schematic structural diagram of a shunt part A-11 according to some embodiments of the present disclosure. The shunt part A-11 shown in FIG. 4 may be applied to the atrial shunt apparatus A-1 shown in FIG. 1.

The view direction of FIG. 2 is a view direction viewed from a left side to a right side of a patient when the patient is in an upright posture. The view direction of FIG. 3 is a view direction viewed from the left side to the right side of the patient when the patient is in a supine posture. In FIG. 2 and FIG. 3, the solid portion of the shunt part A-11 represents a portion arranged in a left atrium of the patient, and the dashed line portion of the shunt part A-11 represents a portion arranged in a right atrium of the patient.

In addition, it should be noted that, in the drawings of the embodiments of the present disclosure, the area where an RA is located is a right atrium of a patient, the area where an LA is located is a left atrium of the patient, the area where an RV is located is a right ventricle of the patient, the area where an LV is located is a left ventricle of the patient, an AS is used for representing an atrial septum, and an H is used for representing a hole at the atrial septum.

It should be understood that, in the present embodiment of the present disclosure, for example, a hole may refer to an opening formed at an atrial septum between a left atrium and a right atrium of a patient through an ostomy. The atrial shunt apparatus may shunt blood from the left atrium to the right atrium through the hole on the atrial septum of the patient.

Referring to FIGs. 1 to 4, the atrial shunt apparatus A-1 may include a shunt part A-11. The shunt part A-11 defines a shunt pathway A-111. The shunt pathway A-111 may be used for communicating with a left atrium and a right atrium of a patient, to shunt blood from the left atrium to the right atrium. The shunt pathway A-111 may have a first opening A-111a and a second opening A-111b.

When the atrial shunt apparatus is mounted in atria of the patient, the shunt pathway A-111 may be configured to communicate with the left atrium and the right atrium through the first opening A-111a and the second opening A-111b respectively. The first opening A-111a may be configured to be closer to a head and/or a front of a body of the patient relative to the second opening A-111b.

If the first opening is closer to the head of the patient relative to the second opening, when the patient is in an upright position (or a posture close to it, such as a sitting posture that an upper body is upright), the first opening A-111a is higher than the second opening A-111b in a direction of gravity, so that blood and/or a suspended substance (such as micro-thrombus) in the right atrium needs to overcome the gravity to reach the left atrium.

If the first opening is closer to the front of the body of the patient relative to the second opening, when the patient is in a supine posture (or a posture close to it), the first opening A-111a is higher than the second opening A-11 1b in the direction of the gravity, so that the blood and/or the suspended substance (such as micro-thrombus) in the right atrium needs to overcome the gravity to reach the left atrium.

If the first opening is closer to the front and the front of the body of the patient relative to the second opening, when the patient is in the upright position and the supine posture (or a posture close to the upright posture or the supine posture), the first opening A-111a is higher than the second opening A-111b in the direction of the gravity, so that the blood and/or the suspended substance (such as micro-thrombus) in the right atrium needs to overcome the gravity to reach the left atrium.

Consequently, when the patient is in the upright posture or the supine posture (or a posture close to the upright posture or the supine posture), the atrial shunt apparatus of the embodiments of the present disclosure effectively prevents or reduces the blood and/or the suspended substance in the blood from entering into the left atrium from the right atrium. Compared with providing a valve, this method may avoid a problem of blockage of a pathway. In daily life, a person will be in the upright posture or the supine posture most of the time (or a posture close to the upright posture or the supine posture). Therefore, the atrial shunt apparatus according to the present embodiment of the present disclosure meets the requirements of a patient for daily activities.

In some embodiments, referring again to FIGs. 1 to 4, when the atrial shunt apparatus A-1 is mounted in an atria of a patient, the first openings A-111a may be configured to be closer to the head of the patient and the front of the body of the patient relative to the hole H, and the second openings A-111b may be configured to be closer to the foot of the patient and the back of the body of the patient relative to the hole H.

In order to more clearly and accurately describe this implementation, a hypothetical plane rectangular coordinate system is constructed, to describe this implementation from another perspective. This coordinate system takes a plane where the atrial septum AS is located as a reference surface, takes a position where the hole H is located as an origin, and, takes an intersection line of a horizontal plane passing through the hole H when the patient is in an upright posture and the plane where the atrial septum AS is located as an X axis, takes a direction from a back to the front of the body of the patient as a positive direction of the X axis, takes a straight line passing through the origin and perpendicular to the X axis, on the reference surface, as a Y axis, and takes a direction from a foot to the head of the body of the patient as a positive direction of the Y axis.

It should be understood that, the atrial septum of the patient is not an ideal plane, the atrial septum has a certain thickness and is not absolutely flat. However, in order to clearly describe the embodiments of the present disclosure as much as possible, it may be considered that the atrial septum is located on a certain plane. For example, the plane where the atrial septum is located may refer to a plane with the highest degree of coincidence with the atrial septum. For example, the plane where the atrial septum is located also may be a fitted plane (or a hypothetical plane), and a sum of distances from all points of the atrial septum to this plane (shortest distance) is minimum.

When the shunt apparatus A-1 is mounted in the atrium of the patient, the first opening A-111a may be configured so that an orthographic projection of the first opening on a plane where the atrial septum is located is located in a first quadrant of a hypothetical coordinate system, and the second opening A-111b may be configured so that an orthographic projection of the second opening on the plane where the atrial septum is located is located in a third quadrant of the coordinate system.

As shown in FIG. 2, when the patient is in an upright posture, the first opening A-111a is higher than the second opening A-111b in a direction of gravity (that is, a direction indicated by the thick arrow in the figure). VD1 is used for indicating a height difference between the first opening A-111a and the second opening A-111b in the direction of the gravity at this moment.

As shown in FIG. 3, when the patient is in a supine posture, the first opening A-111a is also higher than the second opening A-111b in the direction of the gravity (that is, a direction indicated by the thick arrow in the figure), and a VD2 is used for indicating a height difference between the first opening A-111a and the second opening A-111b in the direction of the gravity at this moment.

In this way, the first opening A-111a is made to be closer to the head and the front of the body of the patient relative to the second opening, so that when the patient is in the upright position or the supine posture (or a posture close to the supine or the upright posture), and thus the blood and/or the suspended substance in the blood is effectively prevented from entering into the left atrium from the right atrium.

In some embodiments, referring to FIG. 2 and FIG. 3, when the shunt apparatus A-1 is mounted in the atrium of the patient, the first opening A-111a may be configured so that an angle α, between the X-axis and a connecting line between an orthographic projection of the first opening A-111a on the plane where the atrial septum is located and the hole H, ranges from 30 degrees to 60 degrees, and the second opening A-11 1b may be configured so that an included angle β, between the X-axis and a connecting line between an orthographic projection of the second opening A-1 1 1b on the plane where the atrial septum is located and the hole H, ranges from 30 degrees to 60 degrees. More preferably, the included angle α may be 45 degrees, and the included angle βmay also be 45 degrees.

In this way, when the patient is in the upright and the supine posture (or a posture close to the upright or the supine posture), the height differences VD1 and VD2 between the first opening and the second opening may be made relatively close, thereby ensuring that the shunt apparatus may provide a stable and sufficient resistance when the patient is in the upright posture or the supine posture (or a posture close to the supine posture or the upright posture), to prevent or reduce the blood and/or the suspended substance in the blood from entering into the left atrium from the right atrium.

For the height differences VD1 and VD2, the embodiment of the present disclosure is not specifically limited, and a person skilled in the art may set them based on an actual requirement.

Illustratively, in some embodiments, the height differences VD1 and VD2 may be set to range from 1.5cm to 2cm. This height difference will generate a pressure gradient approximately equal to or higher than 2 mmHg, which is sufficient to prevent or reduce the blood and/or the suspended substance in the blood from entering into the left atrium from the right atrium under most cases. In this way, it is ensured that the shunt apparatus is compact in structure while meeting use requirements.

In some embodiments, referring again to FIG. 2 and FIG. 3, when the shunt apparatus A-1 is mounted in the atrium of the patient, a length of a connecting line TL of orthographic projections of the first opening A-111a and the second opening A-111b on the plane where the atrial septum is located may range from 0.5 cm to 4 cm, and more preferably, the TL may range from 2 cm to 3 cm. The TL is set to be this length range, coordinating with the included angle α and the included angle β, to make the height differences VD1 and VD2 range from 1.5 cm to 2 cm.

In some embodiments, referring again to FIGs. 1 to 4, the shunt part A-11 may be tubular, and the shunt part A-11 includes a first portion pipe body A-11a and a second portion pipe body A-11b. The first portion pipe body A-11a may be configured to be arranged in the left atrium of the patient, and the second portion of the second portion pipe body A-11b may be configured to be arranged in the right atrium of the patient. The first openings A-111a may be disposed on the first portion pipe body A-11a, and the second openings A-111b may be disposed on the second portion pipe body A-11b.

The shunt part A-11 may further include a third portion pipe body A-11c. The third portion pipe body A-11c communicates with the first portion pipe body a-11a and the second portion pipe body A-11b. When the shunt apparatus A-1 is mounted in the atrium of the patient, the third portion pipe body A-11c may be configured to pass through the hole H at the atrial septum.

The first portion pipe body A-11a may be substantially parallel to the second portion pipe body A-11b, and the first portion pipe body A-11a and the second portion pipe body A-11b form an included angle (that is, non-parallel) with the third portion pipe body A-11c respectively. In other words, an axis of the first portion pipe body A-11a and an axis of the second portion pipe body A-11b are substantially parallel and form an included angle with an axis of the third portion pipe body A-11c.

An extending direction of the first portion pipe body A-11a (that is, a direction from an end of the first portion pipe body A-11a close to the third portion of the pipe body A-11c to an end of the first portion pipe body A-11a away from the third portion pipe body A-11c) is opposite to an extending direction of the second portion pipe body A-11b (that is, a direction from an end of the second portion pipe body A-11b close to the third portion pipe bodies A-11c to an end of the second portion pipe body A-11b away from the third portion pipe bodies A-11c). In other words, the first portion pipe body A-11a and the second portion pipe body A-11b may extend along opposite directions from the third portion pipe bodies A-11c, respectively. The shunt part A-11 is integrally constructed in a shape similar to a letter "Z".

During mounting, the first portion pipe body A-11a may be mounted in the left atrium, and the first opening A-111a is made be closer to the front and the head of the body of the patient relative to the hole H. Correspondingly, the second opening A-111b is made be closer to the back and the foot of the body of the patient relative to the hole H.

In this way, when the patient is in the upright or the supine posture (or a posture close to the supine or the upright posture), it may be ensured that the first opening A-111a is higher than the second opening A-111b in the direction of the gravity. Meanwhile, the shunt part is constructed in such a structure that makes the first portion pipe body A-11a and the second portion pipe body A-11b be close to the atrial septum of the patient, so that excessive extension of the shunt part to the middle part of the left atrium and the middle part of the right atrium may be avoided, and thus disturbance of the shunt part to blood flow in the atrium may be reduced, turbulence may be avoided, and thus formation of thrombus may be reduced.

In some embodiments, referring again to FIGs. 1 to 4, an included angle between the first portion pipe body A-11a and the third partial pipe body A-11c may be larger than 90 degrees, for example, may be 135 degrees. An included angle between the second portion pipe body A-11b and the third portion pipe body A-11c may be larger than 90 degrees, for example, may be 135 degrees. In other words, an included angle between an axis of the first portion pipe body A-11a and an axis of the third portion pipe body A-11c is larger than 90 degrees, for example, may be 135 degrees.

The included angle between the first portion pipe body A-11a and the third portion pipe body A-11c and the included angle between the second portion pipe body A-11b and the third portion pipe body A-11c are set to be larger than 90 degrees, to make the pathway of the shunt part is relatively gentle, and the change of the pathway is relatively moderate, to avoid turbulence when the blood flows through the shunt part, and thus the formation of the thrombus is reduced.

In some embodiments, the first opening A-111a may be disposed on a peripheral wall of the first portion pipe body A-11a, and the second opening 112 may be disposed on a peripheral wall of the second portion pipe body A-11b.

In view of a flow manner of the blood in the atrium, the opening is disposed on a peripheral wall of the shunt part, so that the blood may be prevented from being directly injected into the shunt part, and thus the possibility of occurrence of vortex is reduced, and the formation of the thrombus is reduced.

In some embodiments, the first openings A-11 1a may be close to an end of the first portion pipe body A-11a, and the second openings A-111b may be close to an end of the second portion pipe body A-11b. In this way, a distance between the first opening A-111a and the second opening A-111b may be farther, and thus the structure of the shunt part is more compact.

Certainly, it is unnecessary that the first opening A-111a and the second opening A-111b are disposed on the peripheral wall of the pipe body. In other embodiments of the present disclosure, the first opening and the second opening may also be respectively disposed on end faces of the first portion pipe body A-11a and the second portion pipe body A-11b.

FIG. 5 is a schematic structural diagram of an atrial shunt apparatus A-2 configured in atria according to some other embodiments of the present disclosure.

As shown in FIG. 5, the atrial shunt apparatus A-2 is substantially the same as the atrial shunt apparatus A-1. For the purpose of brevity, the similarities will not be repeated herein.

Referring to FIG. 5, in this embodiment, the first opening A-211a and the second opening A-211b of the atrial shunt part A-21 are disposed on end faces of the first portion pipe body A-21a and the second portion pipe body A-21b, respectively.

In view of the flow manner of the blood in the atria, the opening of the shunt part is disposed on the end face, so that the blood may be directly injected into the shunt part, thereby increasing the flow rate at the opening, and thus the formation of blockage and/or hyperplasia caused by the blood or floating substances in the blood adsorbed on the opening is avoided or reduced.

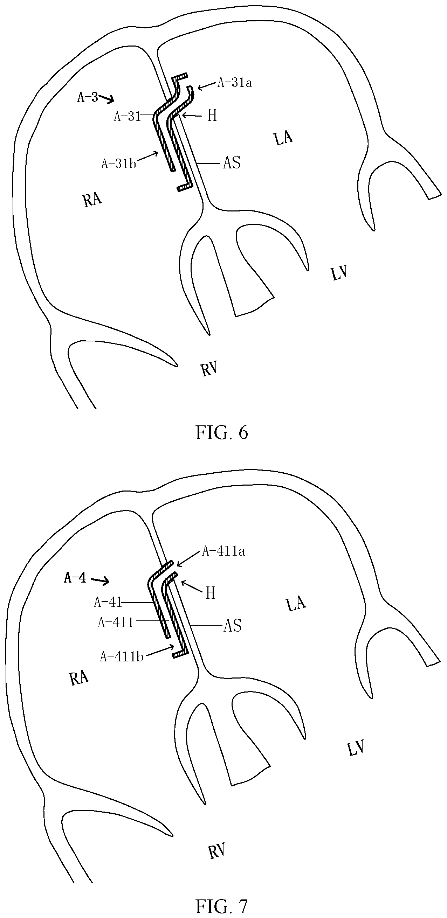

FIG. 6 is a schematic structural diagram of an atrial shunt apparatus A-3 configured in atria according to some other embodiments of the present disclosure.

As shown in FIG. 6, the atrial shunt apparatus A-3 is substantially the same as the atrial shunt apparatus A-1. For the purpose of brevity, the similarities will not be repeated herein.

Referring to FIG. 6, volume of the first portion pipe body A-31a of the shunt part A-31 is less than volume of the second portion pipe body A-31b. In other words, the first portion pipe body A-31a of the shunt part A-31 is shorter than the second portion pipe body A-31b. That is, an axial length of the first portion pipe body A-31a is less than an axial length of the second portion pipe body A-31b.

In view of the flow manner of the blood in the left atrium, due to high pressure of the left atrium, the portion of the shunt part arranged in the left atrium is more prone to cause turbulence, and thus thrombus is induced. Meanwhile, the smaller the volume of the implanted object in the left atrium, the smaller the influence on the blood flow of the left atrium, and the smaller the area that needs endothelialization, which is more conducive to reducing risk of formation of the thrombus in the left atrium. The shunt part is set as the first portion pipe body (that is, the part of the shunt part arranged in the left atrium) is less than the second portion pipe body (that is, the part of the shunt part arranged in the right atrium), so that the possibility of the formation of the thrombus is reduced.

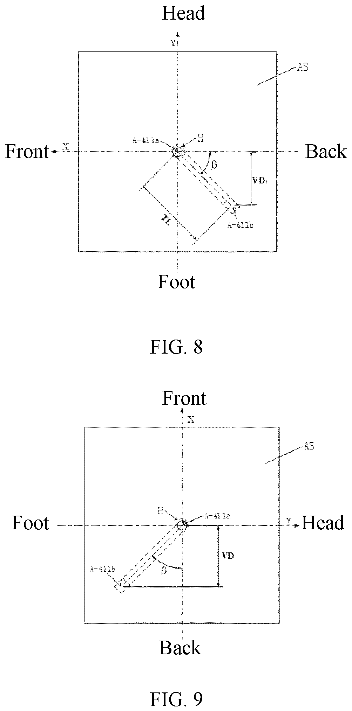

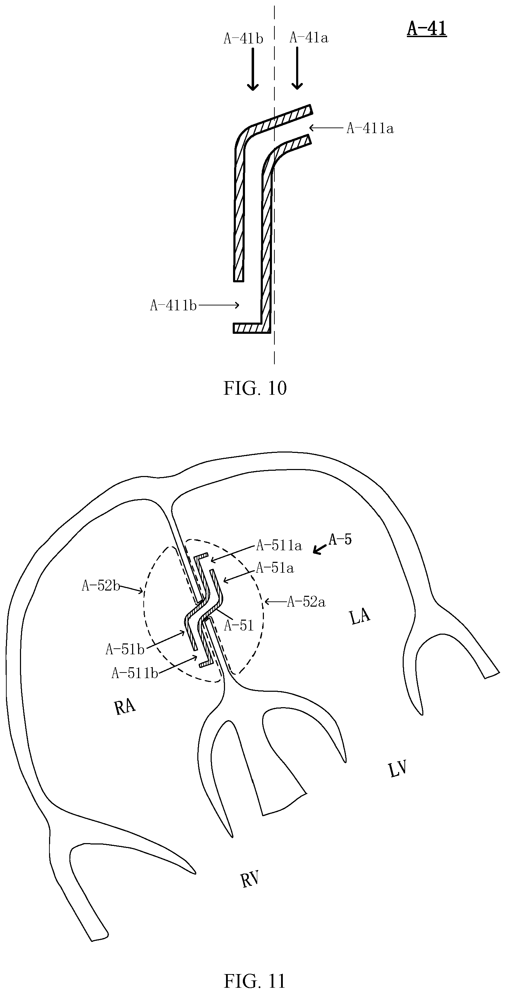

FIG. 7 is a schematic structural diagram of an atrial shunt apparatus A-4 configured in atria according to some other embodiments of the present disclosure. FIG. 8 and FIG. 9 are schematic structural diagrams of other view directions of FIG. 7 in which the atrial shunt apparatus is configured in the atrium. FIG. 10 is a schematic structural diagram of a shunt part according to some embodiments of the present disclosure. The shunt part A-41 shown in FIG. 10 may be applied to the atrial shunt apparatus shown in FIG. 7.

The view direction of FIG. 8 is a view direction viewed from a left side of to a right side of a patient when the patient is in an upright posture. The view direction of FIG. 9 is a view direction viewed from the left side to the right side of the patient when the patient is in a supine posture. In FIG. 8 and FIG. 9, the dashed line is used for indicating the pipe body portion of the shunt part A-41 arranged in the right atrium of the patient.

Referring to FIGs. 7 to 10, the atrial shunt apparatus A-4 includes a shunt part A-41. The shunt part A-41 defines a shunt pathway A-411. When the atrial shunt apparatus A-4 is mounted in the atrium of the patient, the shunt pathway A-411 may be configured to communicate with a left atrium and a right atrium through a first opening A-411a and a second opening A-411b respectively.

When the atrial shunt apparatus A-4 is mounted in the atrium of the patient, the first opening A-411a is configured so that an orthographic projection of the first opening A-411a on a plane where the atrial septum is located is at least partially coincides with the hole H, and the second opening A-411b is configured to be closer to a foot and a back of the body of the patient relative to the hole H.

To put it another way, when the atrial shunt apparatus A-4 is mounted in the atrium of the patient, the first opening A-411a is configured so that the orthographic projection of the first opening on the plane where the atrial septum is located substantially coincides with the origin of the hypothetical coordinate system, and the second opening A-411b is configured so that the orthographic projection of the second opening on the plane where the atrial septum is located is located in the third quadrant of the coordinate system. About specific description of the hypothetical coordinate system, please refer to the aforementioned embodiment, and for the purpose of brevity, it will not be repeated herein.

In this way, the first opening may be closer to the head and the front of the body of the patient relative to the second opening, so that the first opening will be higher than the second opening in the direction of the gravity when the patient is in the upright posture or the supine posture (or a posture close to the upright posture or the supine posture). Meanwhile, in this way, the portion of the shunt part arranged in the left atrium may be further reduced, and thus the surface area that needs endothelialization and the risk of the formation of thrombus is reduced.

In some embodiments, referring again to FIGs. 7 to 10, when the shunt apparatus A-4 is mounted in the atrium of the patient, the first opening A-411a may be configured so that an orthographic projection of the first opening A-411a on the plane where the atrial septum is located is substantially coincident with the hole H, and the second opening A-411b may be configured so that an included angle β, between the X-axis and a connecting line between an orthographic projection of the second opening A-411b on the plane where the atrial septum is located and the hole H, ranges from 30 degrees to 60 degrees. More preferably, the included angle β may be 45 degrees.

In this way, the height differences VD1 and VD2 between the first opening and the second opening may be substantially the same when the patient is in the upright and the supine posture. Consequently, the shunt part may provide more stable resistance at different body positions.

In some embodiments, referring again to FIGs. 7 to 10, the height differences VD1 and VD2 may be set to ranges from 1.5 cm to 2 cm.

In some embodiments, referring again to FIGs. 7 to 10, when the shunt apparatus A-4 is mounted in the atrium of the patient, a length of a connecting line TL between the orthographic projections of the first opening A-411a and the second opening A-411b on the plane where the atrial septum AS is located may range from 0.5 cm to 4 cm, and more preferably, the TL may range from 2 cm to 3 cm.

In some embodiments, referring again to FIGs. 7 to 10, the shunt part A-41 may be tubular, and the shunt part A-41 includes a first portion pipe body A-41a and a second portion pipe body A-41b. An included angle is formed between the first portion pipe body A-41a and the second portion pipe body A-41b (that is, non-parallel), in other words, an included angle is formed between an axis of the first portion pipe body A-41a and an axis of the second portion pipe body A-41b.

When the shunt apparatus A-4 is mounted in the atrium of the patient, the second portion pipe body A-41b may be configured to be arranged in the right atrium, and the first portion pipe body A-41a may be configured to pass through the hole H of the atrial septum and enable an end of the first portion pipe body A-41a to be arranged in the left atrium.

The first opening A-411a may be disposed on an end face of the first portion pipe body A-41a, and the second opening A-411b may be disposed on a peripheral wall of the second portion pipe body A-41b. In other embodiments of the present disclosure, the second openings A-411b may also be disposed on an end face of the second portion pipe body A-41b. The first portion pipe body A-41a may be shorter than the second portion pipe body A-41b, so that merely a small portion of the structure is disposed in the left atrium after the first portion pipe body A-41a passes through the atrial septum AS.

During installation, the first opening A-411a may be arranged in the left atrium, to make merely a small portion of the first portion pipe body A-41a be arranged in the left atrium, so that the projection of the first opening A-411a on the plane where the atrial septum is located substantially coincides with the hole H. The second portion pipe body A-41b is arranged in the right atrium, to make the second opening A-41 1b be closer to the foot and the back of the body of the patient relative to the hole H.

In this way, when the patient is in the upright or the supine posture (or a posture close to the supine posture or the upright posture), the first opening is higher than the second opening in the direction of the gravity. Meanwhile, such an arrangement may cause that the pipe body portion of the shunt part disposed in the left atrium has an extremely small size, and thus turbulence in the left atrium is significantly reduced, and the formation of thrombus is effectively reduced.

In some embodiments, an inner diameter of the shunt part provided in this embodiment of the present disclosure ranges from 4 mm to 12 mm, preferably, ranges from 8 mm to 12 mm, and more preferably, ranges from 10 mm to 12mm.

Since the resistance of the blood when the blood flows through the shunt part is related to the inner diameter of the shunt part, the resistance generated by the shunt part is relatively proper if the inner diameter of the shunt part is set to be in the above-mentioned range.

In some embodiments, an inner wall of the shunt pathway provided in the present embodiment of the present disclosure may be hydrophobic and/or resistant to formation of neoplasma, to prevent or reduce the adsorption of blood or floating objects in the blood on an inner wall of the shunt pathway. In other words, the inner wall of the shunt pathway provided in the present embodiment of the present disclosure may be a surface which is hydrophobic and/or resistant to formation of neoplasma.

In some embodiments, the shunt part provided in the present embodiment of the present disclosure may include a supporting mesh frame and an inner lining pipe. The supporting mesh frame is tubular, and the inner lining pipe is arranged in the supporting mesh frame.

Illustrativelly, the supporting mesh frame may be made of a metal with a relatively high biocompatibility such as nickel-titanium alloy, and a surface of the supporting mesh frame is smooth. The inner lining pipe may be made of a polymer material which is hydrophobic, and/or resistant to formation of neoplasma or anti-tissue endogenous, to prevent the tissue hyperplasia from blocking the pathway.

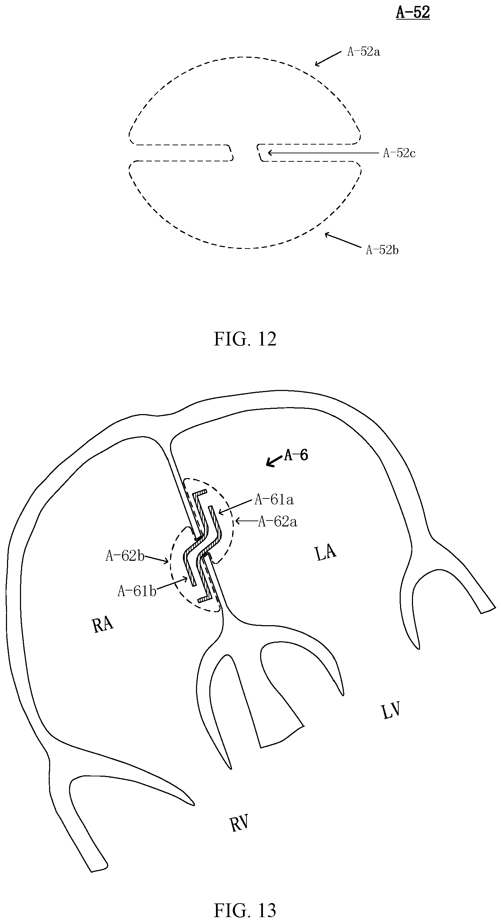

FIG. 11 is a schematic structural diagram of an atrial shunt apparatus A-5 configured in atria according to some other embodiments of the present disclosure. FIG. 12 is a schematic structural diagram of a frame part according to some embodiments of the present disclosure. The shunt part A-52 shown in FIG. 12 may be applied to the atrial shunt apparatus A-5 shown in FIG. 11.

Referring to FIG.11 to FIG.12, besides the shunt part A-51, the atrial shunt apparatus A-5 may further include a frame part A-52. In some embodiments, the frame part A-52 may have a mesh frame. The frame part A-52 may be used for supporting (fixing) the shunt part A-51. In some embodiments, the shunt part A-51 and the frame part A-52 may be two independent components. In some embodiments, the shunt part A-51 and the frame part A-52 may be integrally formed.

The frame part A-52 may include a first frame part A-52a, a second frame part A-52b, and a connecting part A-52c. An end of the connecting part A-52c is connected to the first frame part A-52a, and the other end of the connecting part A-52c is connected to the second frame part A-52b.

When the atrial shunt apparatus A-5 is mounted in the atrium of the patient, the first frame part A-52a may be configured to be arranged in the left atrium of the patient, the second frame part A-52b may be configured to be arranged in the right atrium of the patient, and the connection portion A-52c may be configured to pass through the hole on the atrial septum.

The shunt part A-51 may be tubular, and the shunt part A-51 includes a first portion pipe body A-51a and a second portion pipe body A-51b. A first opening A-511a and a second opening A-511b of the shunt part A-51 are disposed on the first portion pipe body A-51a and the second portion pipe body A-51b, respectively. When the atrial shunt apparatus A-5 is mounted in the atria of the patient, the first portion pipe body A-51a may be configured to be arranged in the left atrium of the patient and surrounded by the first frame part A-52a, and the second portion pipe body A-51b may be configured to be arranged in the right atrium of the patient, and surrounded by the second frame part A-52b.

By providing the grid frame part, the position and posture of the shunt part may be better fixed. In addition, most existing atrial shunt apparatuses adopt a mesh frame, and clinical research shows that this mesh frame is not prone to cause adverse reactions. Therefore, placing the shunt part in the mesh frame may reduce influence of the shunt part on physiological function of the atrium, and the possibility of adverse reactions is reduced.

In some embodiments, referring again to FIG. 11 and FIG. 12, the first frame part A-52a and the second frame part A-52b may be arranged symmetrically. When the atrial shunt apparatus A-5 is mounted in the atrium of the patient, an orthographic projection, of the first frame part A-52a on the plane where the atrial septum is located, covers an orthographic projection of the first portion pipe body A-51a on the plane where the atrial septum is located and an orthographic projection of the second portion pipe body A-51B on the plane where the atrial septum is located. An orthographic projection of the second frame part A-52 B on the plane where the atrial septum is located, covers the orthographic projection of the first portion pipe body A-51a on the plane where the atrial septum is located and the orthographic projection of the second portion pipe body A-51b on the plane where the atrial septum is located.

With this arrangement, the distribution of the structure of the atrial shunt apparatus in the left atrium and the right atrium may be more balanced, so that the position and posture of the atrial shunt apparatus are more stable.

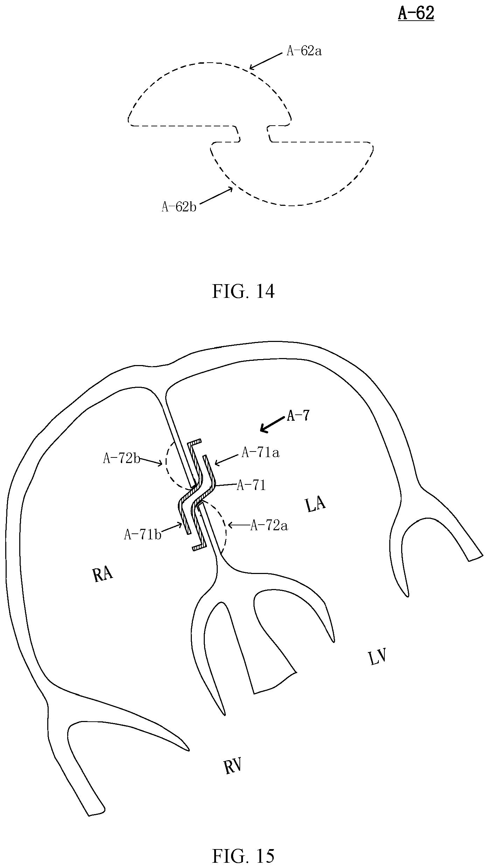

FIG. 13 is a schematic structural diagram of an atrial shunt apparatus A-6 configured in atria according to some other embodiments of the present disclosure. FIG. 14 is a schematic structural diagram of a frame part A-62 according to some other embodiments of the present disclosure. The shunt part A-62 shown in FIG. 14 may be applied to the atrial shunt apparatus A-6 shown in FIG. 13.

As shown in FIG. 13 and FIG. 14, the atrial shunt apparatus A-6 is substantially the same as the atrial shunt apparatus A-5. For the purpose of brevity, the similarities will not be repeated herein.

In the present embodiment, a first frame part A-62a and a second frame part A-62b of the frame part A-62 are arranged asymmetrically (or in a staggered arrangement). In this way, when the atrial shunt apparatus A-6 is mounted in the atria of the patient, an orthographic projection of the second portion pipe body A-61b of the shunt part on the plane where the atrial septum is located is at least partially located outside an orthographic projection of the first frame part A-62a on the plane where the atrial septum is located, and an orthographic projection of the first portion pipe body A-61a on the plane where the atrial septum is located is at least partially located outside an orthographic projection of the second frame part A-62b on the plane where the atrial septum is located. With this arrangement, the first frame part and the second frame part may be downsized, so that the overall structure of the atrial shunt apparatus is more compact.

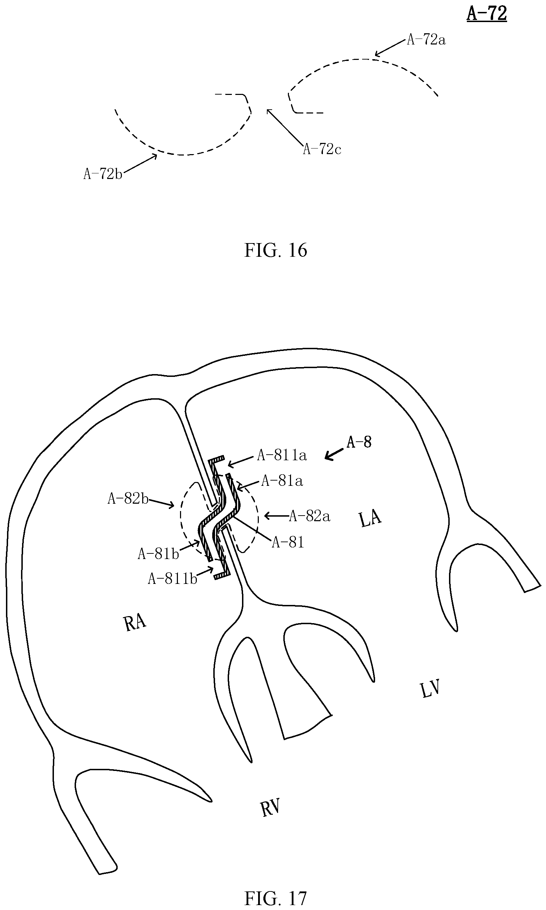

FIG. 15 is a schematic structural diagram of an atrial shunt apparatus A-7 configured in atria according to some other embodiments of the present disclosure. FIG. 16 is a schematic structural diagram of a frame part A-72 according to some other embodiments of the present disclosure. The shunt part A-72 shown in FIG. 16 may be applied to the atrial shunt apparatus A-7 shown in FIG. 15.

In this embodiment, the atrial shunt apparatus A-7 includes a shunt part A-71 and a frame part A-72. The frame part A-72 may include a first frame part A-72a, a second frame part A-72b and a connecting part A-72c. The first frame part A-72a and the second frame part A-72b are dome-shaped. The first frame part A-72a may be mounted in the left atrium of the patient. The second frame part A-72b may be mounted in the right atrium of the patient. An end of the connecting part A-72c is connected to the first frame part A-72a, and the other end of the connecting part A-72c is connected to the second frame part A-72b. The connection A-72c may be mounted to pass through the hole on the atrial septum.

The shunt part A-71 includes a first portion pipe body A-71a and a second portion pipe body A-71b. A first opening and a second opening of the shunt part A-71 may be respectively arranged on the first portion pipe body A-71a and the second portion pipe body A-71b. When the atrial shunt apparatus A-7 is mounted in the atria of the patient, the first portion pipe body A-71a may be configured to be arranged in the left atrium of the patient, and be partially or completely located outside a space surrounded by the first frame part A-72a. The second portion of the second portion pipe body A-71b may be configured to be arranged in the right atrium of the patient, and be partially or completely located outside a space surrounded by the second frame part A-72b.

More specifically, when the atrial shunt apparatus A-7 is mounted in the atria of the patient, the first portion pipe body A-71a may be completely or partially located outside the space surrounded by the first frame part A-72a, and an projection of the first portion pipe body A-71a on the plane where the atrial septum is located may be completely or partially covered by an orthographic projection of the second frame part A-72b on the plane where the atrial septum is located. The second portion pipe body A-71b may be completely or partially located outside the space surrounded by the second frame part A-72b, and a projection of the second portion pipe body A-71b on the plane where the atrial septum is located may be completely or partially covered by an orthographic projection of the first frame part A-72a on the plane where the atrial septum is located.

In this way, the distribution of the structure of the atrial shunt apparatus in the atrium may be more balanced, and thus the stability of the atrial shunt apparatus in the atrium is improved.

FIG. 17 is a schematic structural diagram of an atrial shunt apparatus A-8 configured in atria according to some other embodiments of the present disclosure. The atrial shunt apparatuses A-8 are substantially the same as the atrial apparatuses in the above-described embodiments. For the purpose of brevity, the similarities will not be repeated herein.

A first portion pipe body A-81a of the shunt part A-81 may be configured so that a part of the shunt part A-81 is disposed in the first frame part A-82a, and the other part of the shunt part A-81 extends out of the first frame part A-82a, to make a first opening A-811a be exposed outside the first frame part A-82a. Similarly, a second portion pipe body A-81b may be configured so that a part of the second portion pipe body A-81b is disposed in the second frame part A-82b, and the other part of the second portion pipe body A-81b extends out of the second frame part A-82b, to make a second opening A-811b be exposed outside the second frame part A-82b.

In this way, the blood in the atrium may flow into or out of the shunt pathway directly through the first opening and the second opening without passing through a hole on the frame part, so that a problem that the blood cannot be shunted through the shunt apparatus due to the hole on the frame part being plugged may be avoided.

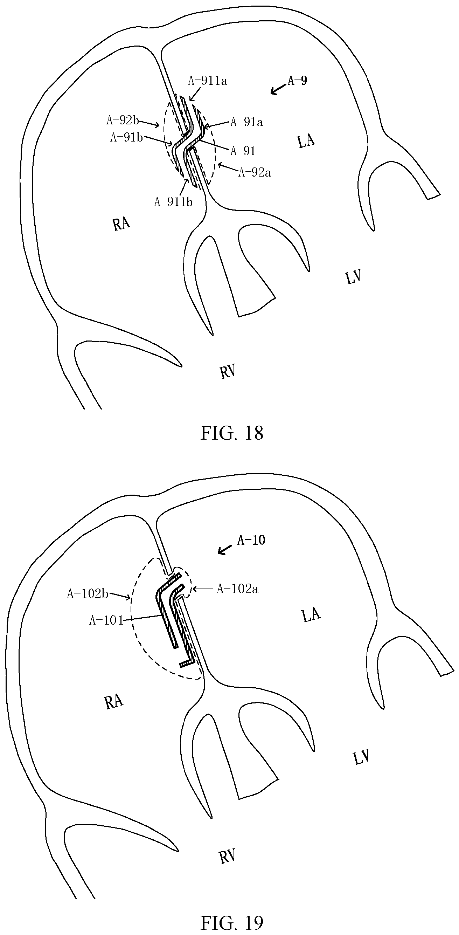

FIG. 18 is a schematic structural diagram of an atrial shunt apparatus A-9 configured in atria according to some other embodiments of the present disclosure. The atrial shunt apparatus A-9 is substantially the same as the atrial shunt apparatus in the above-described embodiments. For the purpose of brevity, the similarities will not be repeated herein.

In this embodiment, a first portion pipe body A-91a of the shunt parts A-91 may be configured to extend from an interior of the first frame part A-92a to a surface of the first frame part A-92a (or a boundary of the first frame part A-92a), and form a first opening A-911a on the surface of the first frame part A-92a. A second portion pipe body A-91b may be configured to extend from an interior of the second frame part A-92b to a surface of the second frame part A-92b, and form a second opening A-911b on the surface of the second frame part A-92b.

In this way, the blood in the atrium may flow into or out of the shunt pathway directly through the first opening and the second opening without passing through a hole on the frame part, so that a problem that the blood cannot be shunted through the shunt apparatus due to the hole on the frame part being plugged may be avoided. In addition, this implementation may further ensure that the shunt part does not protrude out of the frame part, and thus the influence on the flow of the blood is reduced, and the possibility of the formation of thrombus is reduced.

FIG. 19 is a schematic structural diagram of an atrial shunt apparatus A-10 configured in atria according to some other embodiments of the present disclosure. The atrial shunt apparatuses A-10 are substantially the same as the atrial apparatuses in the above-described embodiments. For the purpose of brevity, the similarities will not be repeated herein.

As shown in FIG. 19, a first frame part A-102a of a frame part of the shunt apparatus A-10 may be less than a second frame part A-120b. In other words, a space occupied by the first frame part A-102a is less than a space occupied by the second frame part A-120b.

In view of the flow manner of the blood in the left atrium, due to the higher pressure of the left atrium, the portion of the shunt apparatus arranged in the left atrium is more prone to cause turbulence, and thus the thrombus is induced. Meanwhile, the smaller the volume of the implanted object in the left atrium, the smaller the influence on the blood flow of the left atrium, and the smaller the area that needs endothelialization, which is more conducive to reducing the risk of the formation of the thrombus in the left atrium.

In some embodiments, referring again to FIG. 19, a shunt part A-101 of the shunt apparatus A-10 may adopt the shunt part A-41 in the embodiment shown in FIG. 7, so that the first frame part A-102a may be further reduced.

In some embodiments, the first frame part and the second frame part in the above-mentioned embodiments may be dome-shaped. A surface of the dome-shaped frame part is relatively gentle, so that the influence on the blood flow may be reduced, and thus the possibility of the formation of the thrombus is reduced.

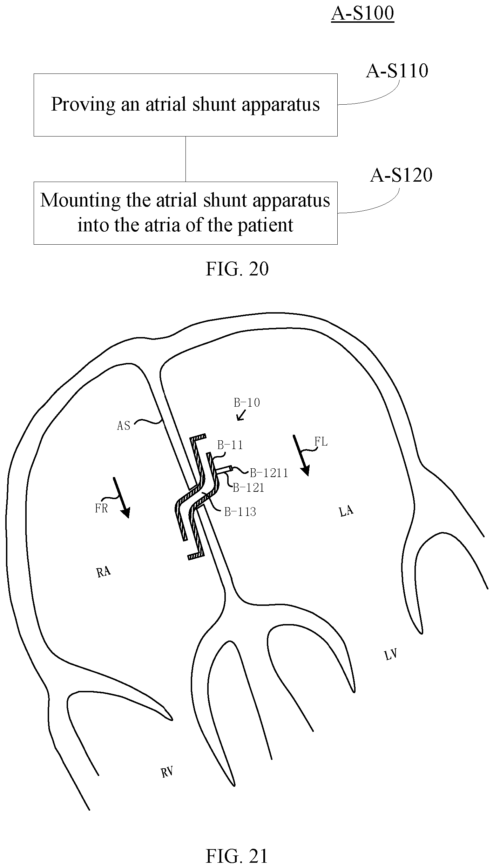

The atrial shunt apparatus provided in the embodiments of the present disclosure is described above with reference to FIGs. 1 to 19. Next, with reference to FIG. 20, the mounting method according to the embodiments of the present disclosure will be illustrated. The mounting method provided in the embodiment of the present disclosure may be used to mount the atrial shunt apparatus provided in the aforementioned embodiment. The embodiments of the method correspond to the embodiments of the apparatus in a one-to-one manner, and for brevity, repeated description is appropriately omitted.

FIG. 20 is a schematic flow diagram of a method for mounting an atrial shunt apparatus A-S100 according to some embodiments of the present disclosure.

Referring to FIG. 20, the method for mounting the atrial shunt apparatus A-S100 includes step A-S 110 and step A-S120.

In step A-S110, providing the atrial shunt apparatus.

The atrial shunt apparatus herein is used for shunting blood from a left atrium to a right atrium through a hole at an atrial septum of a patient. The atrial shunt apparatus includes a shunt part, the shunt part defines a shunt pathway, and the shunt pathway has a first opening and a second opening.

In step A-S120, mounting the atrial shunt apparatus in atria of a patient.

The shunt pathway herein may be configured to communicate with the left atrium and the right atrium through the first opening and the second opening respectively. The first opening is configured to be closer to the head of patient relative to the second opening, and/or the first opening is configured to be closer to the front of the body of patient relative to the second opening.

In other words, the first opening is configured to be higher than the second opening in a direction of gravity when the patient is in an upright posture, and/or the first opening is configured to be higher than the second opening in the direction of the gravity when the patient is in a supine posture.

The atrial shunt apparatus provided in the present disclosure effectively prevents the blood from entering into the left atrium from the right atrium, and there is no problem of pathway blockage.

In some embodiments, the first opening is configured to be closer to a head and a front of a body of the patient relative to the second opening.

In some embodiments, the first opening is configured to be closer to the head and the front of the body of patient relative to the hole, and the second opening is configured to be closer to a foot and a back of the body of the patient.

In some embodiments, the first opening is configured so that an orthographic projection of the first opening on a plane where the atrial septum is located is located in a first quadrant of a hypothetical coordinate system, and the second opening is configured so that an orthographic projection of the second opening on the plane where the atrial septum is located is located in a third quadrant of the coordinate system. The coordinate system is a plane rectangular coordinate system, and the coordinate system takes the plane where the atrial septum is located as a reference surface, takes the hole as an origin, takes an intersection line of a horizontal plane passing through the origin when the patient is in an upright posture and the reference surface as an X axis, takes a direction from a back to the front of the body of the patient as a positive direction of the X axis, takes a straight line passing through the origin and perpendicular to the X axis, on the reference surface, as a Y axis, and takes a direction from a foot to the head of the body of the patient as a positive direction of the Y axis.

In some embodiments, the first opening is configured so that an included angle, between the x axis and a connecting line between an orthographic projection of the first opening on the reference surface and the origin, ranges from 30 degrees to 60 degrees, and the second opening is configured so that an included angle, between the x axis and a connecting line between an orthographic projection of the second opening on the reference surface and the origin, ranges from 30 degrees to 60 degrees.

In some embodiments, the first opening is configured so that the included angle, between the x axis and the connecting line between the orthographic projection of the first opening on the reference surface and the origin, is 45 degrees, and the second opening is configured so that the included angle, between the x axis and the connecting line between the orthographic projection of the second opening on the reference surface and the origin, is 45 degrees.

In some embodiments, the first opening is configured so that an orthographic projection of the first opening on a plane where the atrial septum is located is at least partially coincides with the hole, and the second opening is configured to be closer to a foot and a back of the body of the patient relative to the hole.

In some embodiments, the first opening is configured so that an orthographic projection of the first opening on a plane where the atrial septum is located, substantially coincides with an origin of a hypothetical coordinate system, and the second opening is configured so that, an orthographic projection of the second opening on the plane where the atrial septum is located, is located in a third quadrant of the coordinate system. The coordinate system is a plane rectangular coordinate system, the coordinate system takes the plane where the atrial septum is located as a reference surface, takes the hole as an origin, takes an intersection line of a horizontal plane passing through the origin when the patient is in an upright posture and the reference surface as an X axis, takes a direction from a back to a front of the body of the patient as a positive direction of the X axis, takes a straight line passing through the origin and perpendicular to the X axis, on the reference surface, as a Y axis, and takes a direction from the foot to the head of the body of the patient as a positive direction of the Y axis.

In some embodiments, the second opening is configured so that an included angle, between the x axis and a connecting line between an orthographic projection of the second opening on the reference surface and the origin, ranges from 30 degrees to 60 degrees.

In some embodiments, the second opening is configured so that the included angle, between the x axis and the connecting line between the orthographic projection of the second opening on the reference surface and the origin, is 45 degrees.

In some embodiments, the shunt part is tubular, and the shunt part includes a first portion pipe body and a second portion pipe body. The first portion pipe body is configured to be arranged in the left atrium, and the second portion pipe body is configured to be arranged in the right atrium. The first opening is arranged on an end face or a peripheral wall of the first portion pipe body, and the second opening is arranged on an end face or a peripheral wall of the second portion pipe body.

In some embodiments, the shunt part is tubular, the shunt part includes a first portion pipe body and a second portion pipe body. The first portion pipe body is configured to be arranged in the left atrium, and the second portion pipe body is configured to be arranged in the right atrium. The first opening and the second opening are respectively arranged on the first portion pipe body and the second portion pipe body, and volume of the first portion pipe body is less than volume of the second portion pipe body.

In some embodiments, the shunt part is tubular, and the shunt part includes a first portion pipe body and a second portion pipe body. The first portion pipe body is configured to be arranged in the left atrium, and the second portion pipe body is configured to be arranged in the right atrium. The first opening and the second opening are respectively arranged on the first portion pipe body and the second portion pipe body, and the first portion pipe body is shorter than the second portion pipe body.

In some embodiments, the first opening is configured to be higher than the second opening in a direction of gravity when the patient is in an upright and a supine posture, and a height difference between the first opening and the second opening in the direction of the gravity ranges from 1.5 cm to 2 cm.

In some embodiments, an inner diameter of the shunt pathway ranges from 8 mm to 15 mm.

In some embodiments, an inner wall of the shunt pathway is hydrophobic and/or resistant to formation of neoplasma.

In some embodiments, the atrial shunt apparatus further includes a frame part, and the frame part has a mesh frame. The frame part includes: a first frame part, which is configured to be arranged in the left atrium; a second frame part, which is configured to be arranged in the right atrium; and a connecting part, which connects the first frame part and the second frame part, and is configured to pass through the hole. The shunt part is tubular, and the shunt part includes: a first portion pipe body, where a first opening is arranged on the first portion pipe body, and the first portion pipe body is configured to be surrounded by the first frame part; and a second portion pipe body, where a second opening is arranged on the second portion pipe body, and the second portion pipe body is configured to be surrounded by the second frame part.

In some embodiments, the atrial shunt apparatus further includes a frame part, the frame part includes: a first frame part, which is configured to be arranged in the left atrium; a second frame part, which is configured to be arranged in the right atrium; and a connecting part, which connects the first frame part and the second frame part, and is configured to pass through the hole. The shunt part is tubular, and the shunt part includes: a first portion pipe body, where a first opening is arranged on the first portion pipe body, the first portion pipe body is configured so that a portion of the first portion pipe body is arrange in the first frame part, and the other portion of the first portion pipe body extends out of the first frame part, to make the first opening be exposed out of the frame part; and a second portion pipe body, a second opening is arranged on the second portion pipe body, the second portion pipe body is configured so that a portion of the second portion pipe body is arrange in the second frame part, and the other portion of the second portion pipe body extends out of the second frame part, to make the second opening be exposed out of the frame part.

In some embodiments, the atrial shunt apparatus further includes a frame part, and the frame part includes: a first frame part, which is configured to be arranged in the left atrium; a second frame part, which is configured to be arranged in the right atrium; and a connecting part, which connects the first frame part and the second frame part, and is configured to pass through the hole. The shunt part is tubular, and the shunt part includes: a first portion pipe body, which is configured to extend from an inner of the first frame part to a surface of the first frame part, and form a first opening on the surface of the first frame part; and a second portion pipe body, which is configured to extend from an inner of the second frame part to a surface of the second frame part, and form a second opening on the surface of the second frame part.

In some embodiments, the first frame part is configured so that an orthographic projection of the first frame part on a plane where the atrial septum is located covers an orthographic projection of the first portion pipe body and the second portion pipe body on the plane where the atrial septum is located, and the second frame part is configured so that an orthographic projection of the second frame part on the plane where the atrial septum is located covers the orthographic projection of the first portion pipe body and the second portion pipe body on the plane where the atrial septum is located.

In some embodiments, the first portion pipe body is configured so that an orthographic projection of the first portion pipe body on a plane where the atrial septum is located, is at least partially located outside an orthographic projection of the second frame part on the plane where the atrial septum is located, and the second portion pipe body is configured so that an orthographic projection of the second portion pipe body on the plane where the atrial septum is located, is at least partially located outside an orthographic projection of the first frame part on the plane where the atrial septum is located.

In some embodiments, the first frame part is less than the second frame part. Exemplary atrial implanting device, medical system and method for collecting information of heart

Traditional shunt apparatuses and plugging apparatuses merely can typically achieve atrial shunt or prevent blood flow between the atria for therapeutic purposes, but have no diagnostic function. In order to monitor the health condition of the heart (such as pressure of the atria or condition of heart failure), a patient also needs to be implanted an expensive heart failure diagnosis apparatus separately. This greatly increases the cost of treatment, and the patient (including domestic patients) is generally difficult to accept them or is difficult to burden them.

In view of this, an embodiment of the present disclosure provides an atrial implanting device, which includes a treatment apparatus and a collection apparatus. When applied, the treatment apparatus may be disposed at an opening of an atrial septum of atria of a patient, and the collection apparatus may be disposed on the treatment apparatus to form a combined apparatus.

In some embodiments, the treatment apparatus is used for allowing blood to flow between the left and right atrium of the patient, thereby reducing the pressure of the left atrium by shunting the blood of the left atrium into the right atrium of the patient. In other words, the treatment apparatus is an atrial shunt apparatus for alleviating the continuous high pressure of the left atrium.

In some embodiments, the treatment apparatus is used for preventing blood from flowing between the left and right atrium of the patient. In other words, the treatment apparatus is an atrial septum plugging apparatus for treating disease such as congenital atrial septum defect.

The collecting apparatus is used for collecting activity information of a heart of a patient. For example, the activity information of the heart may include, but is not intended to limit to, one or more of blood pressure information in the heart of the patient, a pressure difference between different heart chambers, blood flow rate information, blood pH information, blood temperature information, blood oxygen information, heart sound information, cardiac contractility information, cardiac acceleration information, cardiac chamber size and change information and intracardiac electrocardiogram information.

Compared with a traditional shunt apparatus, the atrial implanting device provided in the present disclosure may not only achieve the purpose of treatment, but also collect the activity information of the heart of the patient, and thus the monitoring function of the heart working condition is achieved, and is used for helping a doctor to diagnose and treat heart diseases and disease changes. If use the traditional shunt apparatus or plugging apparatus, the patient needs to mount a monitoring apparatus separately, which undoubtedly increases the cost of the treatment, or, the condition change of the patient cannot be known in time, and thus cannot be diagnosed in time. It can be seen therefrom that, the atrial implanting device provided by the present disclosure may provide a therapeutic function for heart disease, such as chronic heart failure, and provide long-term and timely diagnostic information required by a doctor to treat a patient, to better treat and manage the patient, and thus the patient' pain, hospitalization and even mortality are reduced, and the treatment cost is significantly reduced.

The atrial implant apparatus provided by the embodiments of the present disclosure will be illustrated with reference to the drawings below. It should be understood that, there may be many implementations of the present disclosure, and it should not be explained as limitation to the embodiments set forth herein, and the embodiments herein are merely for the more thorough and complete understanding of the present disclosure.

It should be noted that, in the drawings of the embodiments of the present disclosure, an area where an RA is located is the right atrium of the patient, an area where an LA is located is the left atrium of the patient, an area where an RV is located is the right ventricle of the patient, the area where an LV is located is the left ventricle of the patient, an AS is used for representing the atrial septum of the atria, and an H is used for representing an opening at the atrial septum. An arrow FL is used for representing a flow direction of the blood in the left atrium during a diastolic phase (that is, a direction from the pulmonary vein to the mitral valve), and an arrow FR is used for representing the flow direction of the blood in the right atrium during the diastolic phase (that is, a direction from the pulmonary artery to the tricuspid valve).

FIG. 21 illustrates an atrial implanting device B-10 according to an embodiment of the present disclosure.

Referring to FIG. 21, a treatment apparatus of the atrial implanting device B-10 includes a shunt member B-11, and a collection apparatus of the atrial implanting device B-10 includes a hemodynamic information collection unit B-121.

The shunt member B-11 defines a shunt pathway B-113. When the atrial implanting device B-10 is arranged in the atria of the patient, the shunt member B-11 is disposed at an opening of the atrial septum of the patient, and the shunt pathway B-113 communicates with the left atrium and right atrium of the patient, to allow the blood to flow between left atrium and the right atrium of the patient (from a position with high pressure to a position with low pressure).

The hemodynamic information collection unit B-121 is used for collecting hemodynamic information of the heart of the patient. In this embodiment, the hemodynamic information may be blood pressure information in the heart of the patient. Correspondingly, the hemodynamic information collection unit B-121 may include a vibrating diaphragm B-1211 for sensing the pressure information. That is, in this embodiment, the hemodynamic information collection unit B-121 may be a pressure information collection apparatus or a pressure sensor.

The hemodynamic information collection unit B-121 is disposed on an outer wall of the portion of the shunt member B-11 which is located in the left atrium, and the vibrating diaphragm B-1211 is directly exposed to the blood flow in the left atrium.

By setting the shunt member B-11 and the hemodynamic information collection unit B-121, the atrial implanting device B-10 provided in this implementation may not only shunt the blood in the left atrium to the right atrium of the patient to achieve the treatment purpose, but also monitor the pressure information of the blood in the atria of the patient in real time. In addition, the vibrating diaphragm B-1211 is directly exposed to the blood flow, so that the pressure information of the blood may be accurately collected.

It should be understood that, although in the above-mentioned implementation, the hemodynamic information collection unit B-121 is disposed on the outer wall of the portion of the shunt member B-11 that is located in the left atrium, in other embodiments of the present disclosure, the hemodynamic information collection unit B-121 may also be disposed on an outer wall of the portion of the shunt member B-11 that is located in the right atrium, to make the vibrating diaphragm B-1211 is directly exposed to the blood flow in the right atrium.

It should be understood that, in this embodiment of the present disclosure, the hemodynamic information is not limited to blood pressure information in the heart of the patient, and correspondingly, the hemodynamic information collection unit is not limited to a pressure information collection unit. The hemodynamic information may refer to information related to heart motion of a patient and blood flow in the heart, or information directly or indirectly related to hemodynamics of the heart. For example, the hemodynamic information may also be the information of cardiac contractility of the patient, and in this case, the hemodynamic information collection unit may be an accelerometer. For another example, the hemodynamic information may also be blood flow information or blood flow velocity information in the heart of the patient and so on.

It should be understood that, the shape of the hemodynamic information collection unit B-121 is not specifically limited in the present embodiment of the present disclosure. In some embodiments, the hemodynamic information collection units B-121 may be cylindrical, and in other embodiments, the hemodynamic information collection units B-121 may also have other shapes.

It should be understood that, a hemodynamic unit may be integrated with the treatment apparatus as a whole, and it may also be that the treatment apparatus and the collection unit are two organically combined different portions in one apparatus.

In some embodiments, referring again to FIG. 21, when the atrial implanting device B-10 is disposed in the atria of the patient, the vibrating diaphragm B-1211 of the hemodynamic information collection unit B-121 may be configured to be parallel to the atrial septum.

As shown in FIG. 21, the vibrating diaphragm B-1211 of the hemodynamic information collection unit B-121 is configured to be parallel to the atrial septum. In this way, blood flow FL in the left atrium may be prevented from directly impacting the vibrating diaphragm B-1211, so that the influence of the blood flow in the atrium on the collected pressure information may be reduced, and thus the accuracy and comparability of the collected pressure information are improved (relative to different stages or periods).

It should be noted that, although in the embodiment shown in FIG. 21, the hemodynamic information collection unit B-121 is located in the left atrium, in other embodiments of the present disclosure, the hemodynamic information collection unit B-121 may also be configured to be located in the right atrium of the patient and the vibrating diaphragm B-1211 be parallel to the atrial septum, so that blood flow FR in the right atrium may be prevented from directly impacting the vibrating diaphragm B-1211.

In some embodiments, referring again to FIG. 21, the hemodynamic information collection unit B-121 has a first end and a second end opposite to each other, and the vibrating diaphragm B-1211 is disposed on the first end of the hemodynamic information collection unit B-121. When the atrial implanting device B-10 is disposed in the atria of the patient, the hemodynamic information collection unit B-121 may be configured to be perpendicular to the atrial septum from the first end to the second end, and the first end is farther away from the atrial septum relative to the second end.

In this way, the vibrating diaphragm B-1211 of the hemodynamic information collection unit B-121 may be fully exposed to the blood flow in the left atrium, so that the blood flow in the atrium may flush to the vibrating diaphragm B-1211, and thus the "floating object" in the blood is avoided or reduced from being adsorbed on the vibrating diaphragm B-1211.

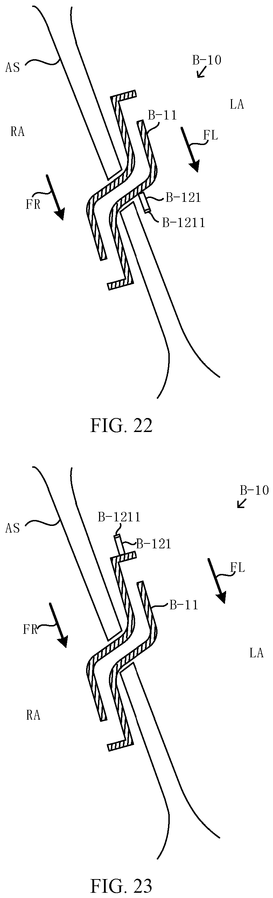

FIG. 22 shows an atrial implanting device according to another embodiment of the present disclosure. The atrial implanting device shown in FIG. 22 is substantially the same as the atrial implanting device shown in FIG. 21. For the purpose of brevity, the similarities will not be repeated herein.

Referring to FIG. 22, the hemodynamic information collection unit B-121 has a first end provided with the vibrating diaphragm B-1211, and a second end opposite to the first end. When the atrial implanting device B-10 is disposed in the atria of the patient, the hemodynamic information collection unit B-121 may be configured so that a direction from the first end to the second end is opposite to the direction of the blood flow FL in the left atrium.

In this way, the blood flow FL in the left atrium may be better prevented from directly impacting the vibrating diaphragm B-1211, so that the influence of the blood flow in the left atrium on the collected pressure information is reduced, and thus the accuracy of the collected pressure information is improved.

It should be noted that, although in the embodiment shown in FIG. 22, the hemodynamic information collection unit B-121 is located in the left atrium, in other embodiments of the present disclosure, the hemodynamic information collection unit B-121 may also be configured to be located in the right atrium, and the direction from the first end to the second end is opposite to the blood flow FR in the right atrium, and thus the blood flow FR in the right atrium is better avoided from directly impacting the vibrating diaphragm B-1211.

FIG. 23 shows an atrial implanting device according to another embodiment of the present disclosure. The atrial implanting device shown in FIG. 23 is substantially the same as the atrial implanting device shown in FIG. 21. For the purpose of brevity, the similarities will not be repeated herein.

Referring to FIG. 23, in this embodiment, when the atrial implanting device B-10 is disposed in the atria of the patient, the hemodynamic information collection unit B-121 may be configured so that a direction from a first end to a second end is the same as the direction of the blood flow FL in the left atrium.

In this way, the blood flow FL in the left atrium may directly impact the vibrating diaphragm B-1211. The blood flow directly impacts the vibrating diaphragm B-1211, so that the floating objects in the blood are avoided or reduced from being adsorbed on the vibrating diaphragm B-1211. In addition, the blood flow FL directly impacts the vibrating diaphragm B-1211, so that the collected pressure information carries related information of blood flow in the left atrium, and thus the hemodynamic condition of the heart of the patient may be analyzed according to the information.

It should be understood that, although in the embodiment shown in FIG. 23, the hemodynamic information collection unit B-121 is located in the left atrium, in other embodiments of the present disclosure, the hemodynamic information collection unit B-121 may also be configured to be located in the right atrium, and the direction from the first end to the second end is the same as the direction of the blood flow FR in the right atrium, so that the blood flow FR in the right atrium directly impacts the vibrating diaphragm, and thus the floating object in the blood is prevented or reduced from being adsorbed on the vibrating diaphragm B-1211, and the collected pressure information carries related information of the blood flow in the right atrium.

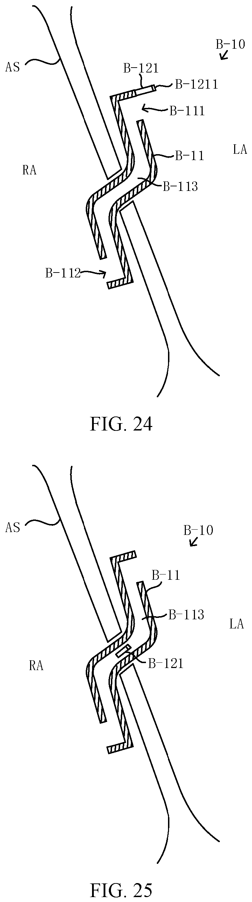

FIG. 24 illustrates an atrial implanting device according to another embodiment of the present disclosure. The atrial implanting device shown in FIG. 24 is substantially the same as the atrial implanting device shown in FIG. 21. For the purpose of brevity, the similarities will not be repeated herein.

Referring to FIG. 24, the shunt member B-11 is provided with a first opening B-111 and a second opening B-112. A shunt pathway B-113 extends from the first opening B-111 to the second opening B-112. When the atrial implanting device B-10 is disposed in the atria of the patient, the first opening B-111 and the second opening B-112 are respectively located in the left atrium and the right atrium of the patient.

In this embodiment, the hemodynamic information collection unit B-121 may be configured to be disposed on an edge of the first opening B-111. In other words, the hemodynamic information collecting unit B-121 may be configured to be disposed near the first opening B-111, to make the blood flowing into the shunting pathway B-113 from the first opening B-111 flush the hemodynamic information collecting unit B-121.

In this way, the blood flowing through the shunt pathway B-113 may flush the hemodynamic information collection units B-121, and thus floating objects in the blood are avoided or reduced from being adsorbed on the hemodynamic information collection units B-121.

It should be noted that, although in the embodiment shown in FIG. 24, the hemodynamic information collection unit B-121 is located at the edge of the first opening B-111, in other embodiments of the present disclosure, the hemodynamic information collection unit B-121 may also be configured to be located at an edge of the second opening B-112. In this way, when the blood flows out from the shunt pathway B-113 via the second openings B-112, the hemodynamic information collection units B-121 may be flushed, and thus the floating objects in the blood are avoided or reduced from being adsorbed on the hemodynamic information collection units B-121.

FIG. 25 illustrates an atrial implanting device according to another embodiment of the present disclosure. The atrial implanting device shown in FIG. 25 is substantially the same as the atrial implanting device shown in FIG. 21. For the purpose of brevity, the similarities will not be repeated herein.

Referring to FIG. 25, in this embodiment, when the atrial implanting device B-10 is disposed in the atria of the patient, the hemodynamic information collection unit B-121 may be configured to be located in the shunt pathway B-113 of the shunt member B-11.

In this way, the blood flowing through the shunt pathway B-113 may continuously scour the hemodynamic information collection units B-121, and thus the floating objects in the blood are avoided or reduced from being adsorbed on the hemodynamic information collection unit B-121.

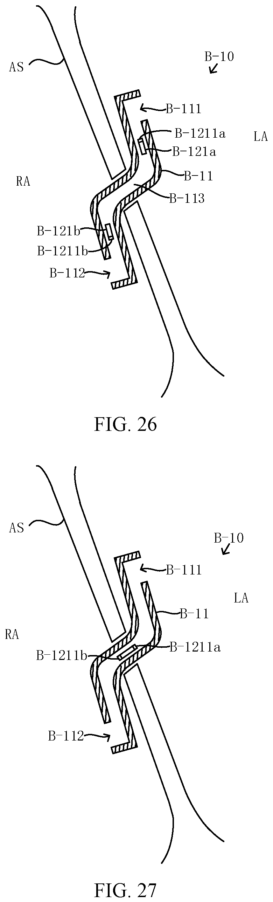

FIG. 26 illustrates an atrial implanting device according to another embodiment of the present disclosure. The atrial implanting device shown in FIG. 26 is substantially the same as the atrial implanting device shown in FIG. 25. For the purpose of brevity, the similarities will not be repeated herein.

Referring to FIG. 26, in this embodiment, the collection apparatus includes two hemodynamic information collection units, that is, a first hemodynamic information collection unit B-121a and a second hemodynamic information collection unit B-121b.

When the atrial implanting device B-10 is disposed in the atria of the patient, the first hemodynamic information collection unit B-121a and the second hemodynamic information collection unit B-121b are configured to be located in the shunt pathway B-113.

In the shunt pathway B-113, in a direction from the left atrium to the right atrium, a vibrating diaphragm B-1211a of the first hemodynamic information collection unit B-121a is located at a upstream side of the first hemodynamic information collection unit B-121a (that is, the upstream side of the first hemodynamic information collection unit B-121a), and a vibrating diaphragm B-1211b of the second hemodynamic information collection unit B-121b is located at a downstream side of the second hemodynamic information collection unit B-121b (that is, the downstream side of the second hemodynamic information collection unit B-121b).

In other words, in the shunt pathway B-113, the vibrating diaphragm B-1211 A is located at a side of the first hemodynamic information collection unit B-121a close to the first opening B-111, and the vibrating diaphragm B-1211b is located at a side of the second hemodynamic information collection unit B-121b close to the second opening B-112.

In this way, the two hemodynamic information collection units B-121a and B-121b may respectively collect the pressure of the blood in the left atrium and the right atrium and the pressure difference information between the left atrium and the right atrium.

FIG. 27 illustrates an atrial implanting device according to another embodiment of the present disclosure. The atrial implanting device shown in FIG. 27 is substantially the same as the atrial implanting device shown in FIG. 26. For the purpose of brevity, the similarities will not be repeated herein.

Referring to FIG. 27, in this embodiment, a housing of the two hemodynamic information collection units is integrally formed. In other words, the two hemodynamic information collection units may share one housing. The two vibrating diaphragms B-1211a and B-1211b are respectively disposed at two sides of this housing. In this way, a space occupied by the two hemodynamic information collection units may be reduced, and the shunting pathway B-113 is prevented from being blocked.

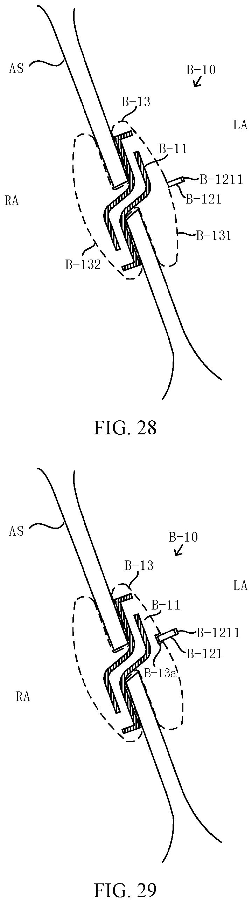

FIG. 28 illustrates an atrial implanting device according to another embodiment of the present disclosure.

Referring to FIG. 28, in this embodiment, the treatment apparatus of the atrial implanting device B-10 further includes a frame B-13 in addition to the shunt member B-11.

When the atrial implanting device B-10 is disposed in the atria of the patient, the frame B-13 is used for fixing the shunt member B-11 at the opening of the atrial septum, to maintain the position and posture of the shunt member B-11.

In some embodiments, the shunt member B-11 and the frame B-13 may be two relatively independent members. In some embodiments, the shunt member B-11 and the frame B-13 may be integrally formed.

In some embodiments, referring again to FIG. 28, the frame B-13 includes a first frame part B-131 and a second frame part B-132. When the atrial implanting device B-10 is disposed in the atria of the patient, the first frame part B-131 and the second frame part B-132 may be configured in the left atrium and the right atrium of the patient, respectively.

As an implementation, the first frame part B-131 and the second frame part B-132 may be dome-shaped.

In an embodiment with a frame, the hemodynamic information collection unit B-121 may be mounted on the frame or on the shunt member. With reference to FIGs. 28 to 30, three possible implementations are provided below.

Referring to FIG. 28, in this embodiment, the hemodynamic information collection unit B-121 is disposed on an outer surface of the frame B-13, to extend the vibrating diaphragm B-1211 into the blood of the atria as much as possible to ensure that the vibrating diaphragm B-1211 may be directly exposed to the blood flow in the atria.

It should be understood that, in other embodiments, the hemodynamic information collection unit B-121 may also be partially located in the frame B-13, as long as the vibrating diaphragm B-1211 is located outside the frame B-13. For example, the shortest distance between the vibrating diaphragm B-1211 and the surface of the frame B-13 may range, but is not limited to, from 1.5 mm to 2.5 mm.

As an example, referring to FIG. 29, in this embodiment, the frame B-13 may be provided with a concave part B-13a for mounting the hemodynamic information collection unit B-121. A part of the hemodynamic information collection unit B-121 may be disposed in the concave part 13a, and a part of the hemodynamic information collection unit B-121 provided with the sensing diaphragm B-1211 may be located outside the concave part B-13a, to ensure that the vibrating diaphragm B-1211 may be directly exposed to the blood flow in the atria.

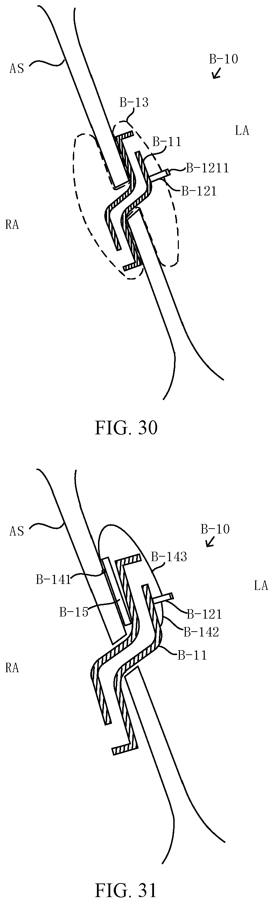

As another example, referring to FIG. 30, in this embodiment, the hemodynamic information collection unit B-121 may be disposed on an outer wall of the shunt member B-11, and a part of the hemodynamic information collection unit B-121 provided with the vibrating diaphragm B-1211 may extend out of the frame B-13, to ensure that the vibrating diaphragm B-1211 may be directly exposed to the blood flow in the atria.

It should be noted that, FIGs. 28 to 30 merely show several possible implementations, rather than all the implementations.

For example, although the hemodynamic information collection unit B-121 is located in the left atrium of the patient in the embodiments of FIGs. 28 to 30, in other embodiments of the present disclosure, the hemodynamic information collection unit B-121 may also be located in the right atrium of the patient.

For another example, in some embodiments, the vibrating diaphragm B-1211 of the hemodynamic information collection unit B-121 may be configured to be parallel to the atrial septum, or the hemodynamic information collection unit B-121 may be configured so that a direction from a first end (that is, the end provided with the vibrating diaphragm B-1211) to a second end (that is, the end opposite to the first end) is the same as or opposite to a direction of the blood flow.

For another example, in some embodiments, the hemodynamic information collection unit B-121 may be configured to be all located in a space surrounded by the frame B-13 or located in a shunt pathway of the shunt member, to prevent the vibrating diaphragm B-1211 from being directly exposed to the blood flow in the left atrium or the right atrium.

FIG. 31 illustrates an atrial implanting device according to another embodiment of the present disclosure.

Referring to FIG. 31, in this embodiment, in addition to a hemodynamic information collection unit B-121, the collection apparatus further includes an intracardiac electrocardiogram collection unit. The intracardiac electrocardiogram collection unit is configured to collect intracardiac electrocardiogram information of a patient.

The intracardiac electrocardiogram collection unit includes a first electrode and a second electrode. Two portions of a housing of the hemodynamic information collection unit B-121 which are insulated from each other constitute a first electrode and a second electrode respectively.

For another example, in some embodiments, the intracardiac electrocardiogram collection unit includes a first electrode and a second electrode. All or a part of the housing of the hemodynamic information collection unit B-121 constitutes the first electrode; and the frame which is insulated from the hemodynamic information collection unit housing constitutes the second electrode.

The intracardiac electrocardiogram collection unit includes a first electrode and a second electrode B-141. A part or all of the housing of the hemodynamic information collection unit B-121 constitutes the second electrode B-141. In other words, the first electrode and the shunt member B-11 are integrally formed. The second electrode B-141 is configured to be in contact with the atrial septum.

The main portions of the intracardiac electrocardiogram collection unit, for example, an energy supply element, a signal processing element and a storage element and the like, may be accommodated in the housing of the hemodynamic information collection unit B-121. In other words, the housings of the intracardiac electrocardiogram collection unit and the hemodynamic information collection unit B-121 may be integrally formed. The main portions of the intracardiac electrocardiogram collection unit may be electrically connected to the first electrode and the second electrode B-141 through a first line B-142 and a second line B-143, respectively. Alternatively, the first electrode may be the housing or a part of the housing or.

The atrial implanting device B-10 further includes a spacer B-15. The spacer B-15 is used for electrically spacing the second electrode B-141 and the shunt member B-11, to prevent the first electrode and the second electrode B-141 from shorting.

Since it has the hemodynamic information collection unit B-121 and the intracardiac electrocardiogram collection unit, the atrial implanting device B-10 provided in this embodiment may monitor both intracardiac blood pressure information and intracardiac electrocardiogram information of the patient. In addition, the electrode of the intracardiac electrocardiogram collection unit and the housing of the hemodynamic information collection unit B-121 are integrally formed, which simplifies the structure, and avoids or reduces the adsorption of the floating objects in the blood.

It should be noted that, FIG. 31 merely shows one exemplary implementation of the intracardiac electrocardiogram collection unit. In other embodiments of the present disclosure, the intracardiac electrocardiogram collection unit may also be implemented in other manners.

For example, in an embodiment providing a shunt member, the shunt member may include a first portion and a second portion which are electrically separated from each other. A first electrode of the intracardiac electrocardiogram collection unit may be formed by the first portion of the shunt member. A second electrode of the intracardiac electrocardiogram collection unit may be formed by the second portion of the shunt member. In other words, the first electrode and the second electrode of the intracardiac electrocardiogram collection unit may be, integrally formed with the first portion and the second portion of the shunt part insulated from each other, respectively.

For another example, in an embodiment providing a hemodynamic information collection unit, a housing of the hemodynamic information collection unit may include a first portion housing and a second portion housing which are electrically separated from each other. A first electrode and a second electrode of the intracardiac electrocardiogram collection unit may be respectively formed by the first portion housing and the second portion housing of the hemodynamic information collection unit which are insulated from each other. In other words, the first electrode and the second electrode of the intracardiac electrocardiogram collection apparatus may be integrally formed with the first portion housing and the second portion housing of the pressure collection apparatus, respectively.

In this way, the structure of the intracardiac electrocardiogram collection unit may be further simplified, and even without providing a connection line for connecting the electrode outside the housing of the hemodynamic information collection unit, and thus the structure of the implanting apparatus is simplified, or even the volume is reduced.

For another example, in an embodiment providing a frame, a first electrode of the intracardiac electrocardiogram collection unit may be formed by a part or all of the frame. In other words, the first electrode of the intracardiac electrocardiogram collection unit may be integrally formed with the frame.

For another example, in an embodiment in which the frame includes a first frame part and a second frame part, the first electrode and the second electrode of the intracardiac electrocardiogram collection unit may be respectively formed by a first frame part and a second frame part. In other words, the first electrode and the second electrode of the intracardiac electrocardiogram collection apparatus may be integrally formed with the first frame part and the second frame part, respectively.

For another example, in some embodiments, the intracardiac electrocardiogram collection unit and the hemodynamic information collection unit may be respectively accommodated in two mutually independent housings.

The electrode of the intracardiac electrocardiogram collection unit is integrally formed with other components (for example, the housing, the shunt member or the frame of the hemodynamic information collection unit), so that the structure of the implanting apparatus is simplified, and even the volume is reduced.

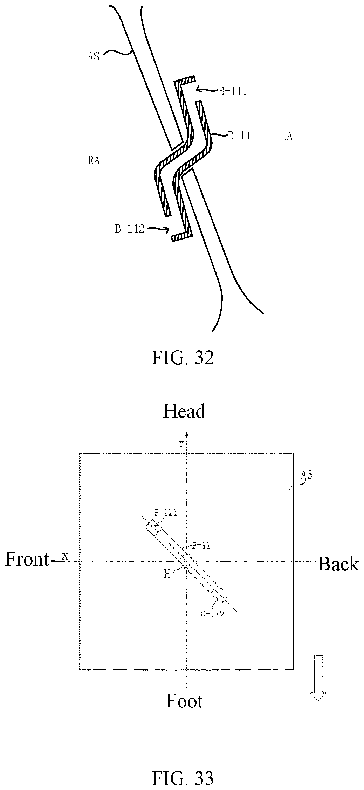

FIG. 32 is a schematic diagram of a shunt member B-11 configured in atria of a patient according to an embodiment of the present disclosure. The shunt member B-11 shown in FIG. 32 may be applied to the atrial implanting device B-10 in the present embodiment of the present disclosure.

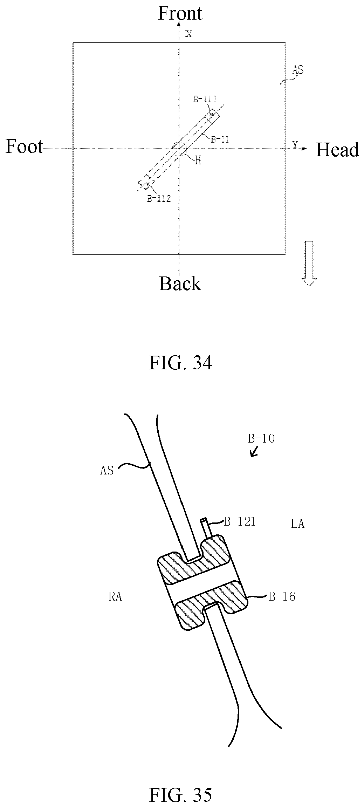

FIG. 33 and FIG. 34 are schematic diagrams of the shunt member B-11 in FIG. 32 from other view directions. The view direction of FIG. 33 is a view direction viewed from a left side to a right of the patient when the patient is in an upright posture. The view direction of FIG. 34 is a view direction viewed from the left side to the right of the patient when the patient is in a supine posture. In FIG.33 and FIG.34, the solid line portion of the shunt member B-11 represents the portion placed in the left atrium of the patient, and the dashed line portion represents the portion placed in the right atrium of the patient.

Referring to FIGs.32 to 34, in this embodiment, a first opening B-111 may be configured to be closer to the front and the head of the body of the patient relative to a second opening B-112 after mounting the shunt member B-11 in the atria of the patient.

If the first opening B-111 is closer to the head and the front of the body of the patient relative to the second opening B-112, the first opening B-111 is higher than the second opening B-112 in a direction of gravity when the patient is in an upright posture and a supine posture (or a posture close to the upright posture or the supine posture), so that the blood and/or suspended substance (such as micro-thrombus) in the right atrium needs to overcome the gravity to reach the left atrium.

Consequently, when a patient is in an upright posture or a supine posture (or a posture close to the upright posture or the supine posture), the shunt member B-11 provided in the present embodiment effectively prevents or reduces the suspended substance in blood and/or blood from entering the left atrium from the right atrium. Compared with setting a valve, in this way, the problem of pathway blockage may be avoided. In daily life, a person is in an upright posture or a supine posture in most of the time (or a posture close to the upright posture or the supine posture). Therefore, the shunt member B-11 provided in this embodiment meets the needs of the patient for daily activities.

In some embodiments, referring again to FIGs.32 to 34, when the shunt member B-11 is disposed in the atria the of the patient, the first opening B-111 may be configured to be closer to the head and the front of the body of the patient relative to the hole H at the atrial septum, and the second opening B-112 may be configured to be closer to the foot and the back of the body of the patient relative to the hole H at the atrial septum.

In order to more clearly and accurately describe this implementation, a hypothetical plane rectangular coordinate system is constructed, to describe the implementation from another perspective. This coordinate system takes a plane where the atrial septum AS is located as a reference surface, takes a position where the hole H is located as an origin, takes an intersection line of a horizontal plane passing through the hole H when the patient is in a upright posture and the plane where the atrial septum AS is located as an X axis, takes a direction from a back to a front of the body of the patient as a positive direction of the X axis, takes a straight line passing through the origin and perpendicular to the X axis, on the reference surface, as a Y axis, and takes a direction from the foot to the head of the body of the patient as a positive direction of the Y axis.

It should be understood that, the atrial septum of the patient is not an ideal plane, it has a certain thickness and is not absolutely flat. However, in order to clearly describe the embodiments of the present disclosure as much as possible, it may be considered that the atrial septum is located on a certain plane. For example, the plane where the atrial septum is located may refer to a plane with the highest degree of coincidence with the atrial septum. For example, the plane where the atrial septum is located may also be a fitted plane (or a hypothetical plane), and a sum of distances from all points of the atrial septum to the plane (shortest distance) is minimum.

When the shunt member B-11 is disposed in the atria of the patient, the first opening B-111 may be configured so that an orthographic projection of the first opening B-111 on the plane where the atrial septum is located is located in a first quadrant of the coordinate system, and the second opening B-112 may be configured so that an orthographic projection of the second opening B-112 on the plane where the atrial septum is located is located in a third quadrant of the coordinate system.

As shown in FIG. 33, when the patient is in an upright posture, the first opening B-111 is higher than the second opening B-112 in a direction of the gravity (that is, the direction indicated by a thick arrow in the figure).

As shown in FIG. 34, when the patient is in a supine posture, the first opening B-111 is also higher than the second opening B-112 in the direction of the gravity (that is, the direction indicated by a thick arrow in the figure).

It can be seen, in this manner, the first opening B-111 may be closer to the head and the front of the body of the patient relative to the second opening B-112, so that when the patient is in an upright or a supine posture (or a posture close to the upright or the supine posture), the blood and/or the suspended substance in the blood is effectively prevented or reduced from entering into the left atrium from the right atrium.

It should be noted that, in the atrial implanting device provided in the embodiment of the present disclosure, the shunt apparatus is not limited to the aforementioned implementations. As an example, FIG. 35 shows an atrial implanting device according to another embodiment of the present disclosure. As shown in FIG. 35, in this embodiment, the atrial implanting device B-10 includes a shunt apparatus B-16, and the hemodynamic information collection unit B-121 is disposed on the shunt apparatus B-16. The shunt apparatus B-16 may be an atrial shunt apparatus provided in the related art.

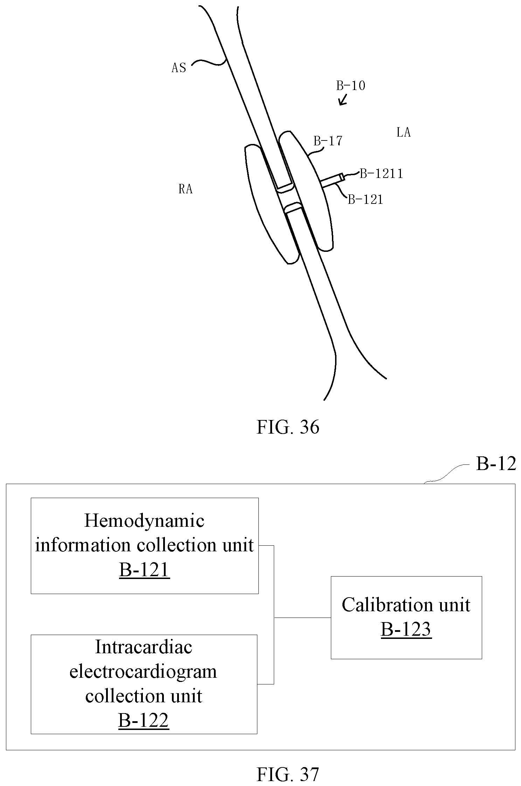

FIG. 36 illustrates an atrial implanting device according to another embodiment of the present disclosure.

Referring to FIG. 36, in this embodiment, the treatment apparatus of the atrial implanting device B-10 may include a plugging member B-17. The plugging member B-17 is used for plugging an opening in atrial septum of the patient to prevent the blood from flowing between the left atrium and the right atrium.

A hemodynamic information collection unit B-121 is mounted on the plugging member B-17, to collect hemodynamic information in the atria of the patient.

With the plugging member B-17 and the hemodynamic information collection unit B-121, the atrial implanting device B-10 provided in this implementation prevents the blood from flowing between the left atrium and the right atrium of the patient to achieve the purpose of treatment, and the purpose of monitoring the hemodynamic information in the atria of the patient is also monitored.

It should be understood that, although in the embodiment shown in FIG. 35, the hemodynamic information collection unit B-121 is disposed on a portion of the plugging member B-17 located in the left atrium, in other embodiments of the present disclosure, the hemodynamic information collection unit B-121may also be disposed at a portion of the plugging member B-17 located in the right atrium.

FIG. 37 shows a collection apparatus according to an embodiment of the present disclosure.

Referring to FIG. 37, in this embodiment, in addition to the hemodynamic information collection unit B-121 and the intracardiac electrocardiogram collection unit B-122, the collection apparatus B-12 further includes a calibration unit B-123.

The calibration unit B-123 is used for simultaneously sending calibration information to the hemodynamic information collection unit B-121 and the intracardiac electrocardiogram collection unit B-122. The hemodynamic information collection unit B-121 and the intracardiac electrocardiogram collection unit B-122 respectively (synchronously) write (combine) time calibration information into the collected hemodynamic information and intracardiac electrocardiogram information.

The time calibration information may be one square wave or a series of square waves sent out at a certain time with a certain amplitude and pulse width. Certainly, it may also be other types of signals that are capable of playing a calibration role.

The calibration unit B-123 may send a time calibration signal to the hemodynamic information collection unit B-121 and the intracardiac electrocardiogram collection unit B-122 at the beginning of collection and/or after finishing collection, or may intermittently send the time calibration signal during the collection process.

One of the effects of the calibration information written into the pressure information and the intracardiac electrocardiogram information is that it may be used as a time stamp. Based on this time stamp, the collected hemodynamic information and the intracardiac electrocardiogram information may be aligned at time, to eliminate a potential time difference between the two, to obtain an accurate corresponding relationship between the two.

The corresponding relationship between the hemodynamic information and the intracardiac electrocardiogram information at time may reflect the hemodynamic condition in a cardiac cycle of the patient more and more accurately. By adopting this implementation, the corresponding relationship between the hemodynamic information and the intracardiac electrocardiogram information at time may be accurately obtained, so that the health condition of the heart of the patient may be better monitored.

An embodiment of the present disclosure further provides a medical system. The medical system provided by an embodiment of the present disclosure will be illustrated with reference to the drawings below.

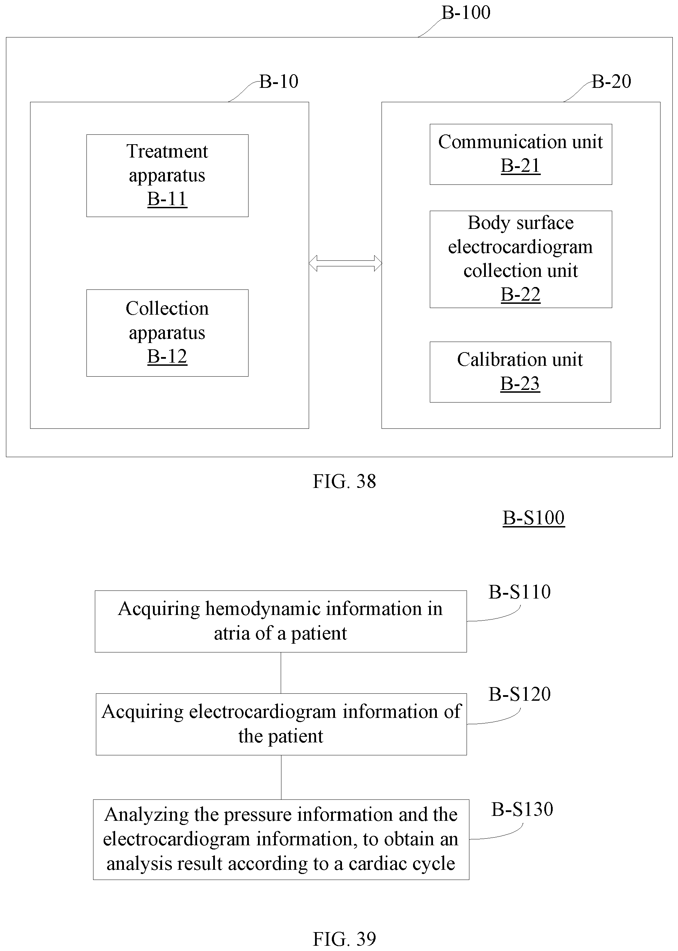

FIG. 38 illustrates a medical system according to an embodiment of the present disclosure.

Referring to FIG. 38, the medical system B-100 includes an atrial implanting device B-10 and an external device B-20. The atrial implanting device B-10 may be any of the atrial implanting devices in the above-mentioned embodiments. In practical applications, the external device B-20 is configured outside the body of the patient.

The external device B-20 includes a communication unit B-21. The communication unit B-21 may be in communication with the atrial implanting device B-10, so that the external device B-20 receive real-time and/or stored activity information, of the heart of the patient collected by the collection apparatus B-12 of the atrial implanting device B-10, through the communication unit B-21.

Optionally, the communication unit B-21 may also be in communication with an external network. The external device B-20 may further include a storage unit, and the storage unit may be used for storing data collected and/or processed by the medical system B-100. The external device B-20 may further include a control unit, and the control unit may be used for sending a control parameter to the atrial implanting device B-10 through the communication unit, to achieve a better function of collecting information by the collection apparatus.

In some embodiments, referring again to FIG. 38, the external device B-20 may further include a body surface electrocardiogram collection unit B-22. The body surface electrocardiogram collection unit B-22 is used for collecting body surface electrocardiogram information of the patient.

The storage unit may also have a computing capability, to determine the health state of the heart of the patient more accurately in combination with the body surface electrocardiogram information and the hemodynamic information in the heart and/or intracardiac electrocardiogram information, and store a processing result, and/or transmit the processing result out through the communication unit, and/or notify a doctor and the patient about a diagnosis result represented by the processing result.

In some embodiments, referring again to FIG. 38, the external device B-20 may further include a calibration unit B-23.

In an embodiment where the collection apparatus B-12 includes merely the hemodynamic information collection unit B-121, the calibration unit B-23 may simultaneously send time calibration information to the hemodynamic information collection unit B-121 and the body surface electrocardiogram collection unit B-22, to make the hemodynamic information collection unit B-121 and the body surface electrocardiogram collection unit B-22 respectively write the time calibration signal into the collected pressure information and body surface electrocardiogram information.

In the embodiments where the collection apparatus B-12 includes the hemodynamic information collection unit B-121 and the intracardiac electrocardiogram collection unit, the calibration unit B-23 may simultaneously send the time calibration information to the hemodynamic information collection unit B-121, the intracardiac electrocardiogram collection unit B-122 and the body surface electrocardiogram collection unit B-22, to make the hemodynamic information collection unit B-121, the intracardiac electrocardiogram collection unit B-122 and the body surface electrocardiogram collection unit B-22 respectively write the time calibration signal into the collected pressure information, the intracardiac electrocardiogram information and the body surface electrocardiogram information.

In some embodiments, the external device may further include an energy supply unit. The collection apparatus of the atrial implanting device may include a receiving coil. The energy supply unit may be configured to cause the receiving coil to generate current, for example, by electromagnetic induction, to provide electric energy for the collection apparatus.

It should be understood that, in other embodiments, the external device may not include an energy supply unit, and the atrial implanting device may be equipped with a battery.

An embodiment of the present disclosure further provides a method for collecting activity information of a heart. The method for collecting the activity information of the heart provided in the embodiments of the present disclosure is illustrated below with reference to the drawings.

FIG. 39 is a schematic flow diagram of a method for collecting activity information of a heart according to an embodiment of the present disclosure. The method for collecting the activity information of the heart may be implemented by the atrial implanting device or the medical system provided in the aforementioned embodiments.

Referring to FIG. 39, the method for collecting the activity information of the heart B-S100 includes steps B-S110 to B-S130.

In step B-S110, acquiring hemodynamic information in atria of a patient.

In steps B-S120, acquiring electrocardiogram information of the patient.

In some embodiments, the electrocardiogram information may be intracardiac electrocardiogram information of the patient. In some embodiments, the electrocardiogram information may be body surface electrocardiogram information. In some embodiments, the electrocardiogram information may include the intracardiac electrocardiogram information and/or the body surface electrocardiogram information of the patient.

In steps B-S130, analyzing the pressure information and the electrocardiogram information, to obtain an analysis result according to a cardiac cycle.

In combination with the pressure information and the electrocardiogram information, the health condition of the heart of the patient may be determined more accurately.

An embodiment of the present disclosure further provides an apparatus for collecting activity information of a heart.

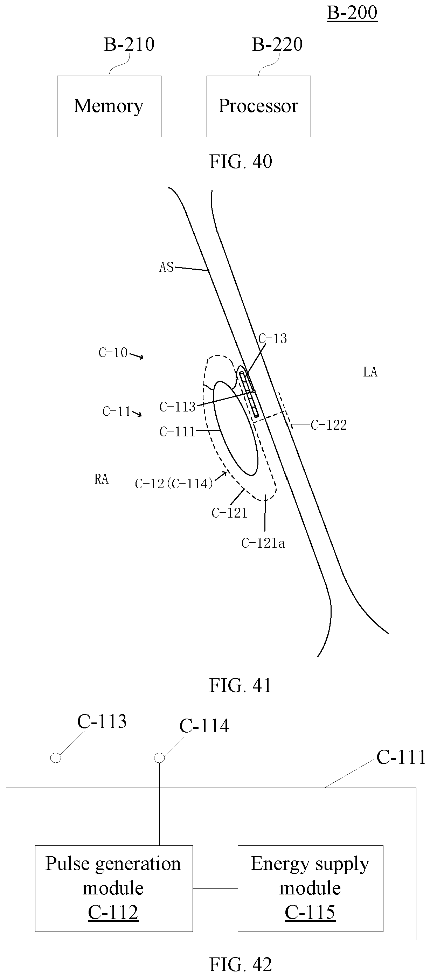

FIG. 40 shows an apparatus for collecting activity information of a heart according to an embodiment of the present disclosure.

Referring to FIG.40, the apparatus for collecting the activity information of the heart B-200 includes a memory B-210 and a processor B-220 coupled to the memory B-210. The processor B-220 is configured to execute the method for collecting the activity information of the heart in the aforementioned embodiment based on an instruction stored in the memory B-210.

### Exemplary Atrial implanting device

FIG. 41 is a schematic diagram of an atrial implanting device C-10 according to an embodiment of the present disclosure. FIG. 42 is a schematic diagram of a pacemaking apparatus C-11 according to an embodiment of the present disclosure.

It should be noted that, in the drawings of the embodiments of the present disclosure, an RA is used for representing a right atrium of a patient, an LA is used for representing a left atrium of the patient, and an AS is used for representing an atrial septum of the patient.

Referring to FIG.41 and FIG.42, the atrial implanting device C-10 includes the pacemaking apparatus C-11 and an anchoring apparatus C-12. The pacemaking apparatus C-11 and the anchoring apparatus C-12 are configured to be implanted in the atria of the patient.

The pacemaking apparatus C-11 includes a housing C-111, a pulse generation module C-112, and a first electrode C-113. The pulse generation module C-112 is accommodated in the housing C-111. The pulse generation module C-112 is used for sensing electrocardiogram, and generating and sending a pacemaking pulse. The first electrode C-113 is configured to be in contact with the atrial septum of the patient. The first electrode C-113 and the second electrode form a sensing and pacemaking loop. The sensing module senses the electrocardiogram activity of the atrial septum through this loop, and the pacemaking pulse generated by the pulse generation module C-112 is delivered to the atrial septum of the patient through this loop.

The anchoring apparatus C-12 is configured to be positioned at the atrial septum AS of the patient, and anchor the housing C-111 of the pacemaking apparatus C-11 to the atrial septum AS of the patient.

The atrial implanting device provided by the present disclosure anchors the housing of the pacemaking apparatus to the atrial septum of the patient by using the anchoring apparatus, and delivers the pacemaking pulse directly to the atrial septum of the patient by using the electrode in contact with the atrial septum. In this way, atrial pacing is achieved, and the pacing effect is better.

In some embodiments, referring to FIG. 41, the anchoring apparatus C-12 is configured to penetrate through the atrial septum of the patient, and has a first portion C-121 located in the right atrium and a second portion C-122 located in the left atrium. The first portion C-121 forms an accommodation space C-121a, and the housing C-111 is accommodated in the accommodation space C-121a. The first electrode C-113 may also directly contact the atrial septum at a left side of the atrial septum, and an isolation layer C-13 may be located between the atrial septum and the second part C-122, to electrically separate the first electrode C-113 from the anchoring apparatus C-12. The isolation layer C-13 may also be a portion of the second portion C-122, such as a covering having an electrically insulation property on its surface.

In this manner, the housing of the pacemaking apparatus may be anchored to the atrial septum of the patient by the anchoring apparatus.

It should be understood that, although in the above-mentioned embodiment, the anchoring apparatus forms the accommodating space, and the housing of the pacemaking apparatus is accommodated in this accommodating space, in other embodiments, the anchoring apparatus may not form the accommodating space. For example, in some embodiments, the housing of the pacemaking apparatus may be connected together with the anchoring apparatus by a fastener.

In some embodiments, referring to FIG. 41 and FIG. 42, the pacemaking apparatus C-11 further includes a second electrode C-114. Illustratively, the first electrode C-113 may be a cathode, and the second electrode C-114 may be an anode. The first electrode C-113 may be directly and stably maintained in contact with the atrial septum (such as through the pressure of the spacer C-13, this pressure may be generated directly from the spacer C-13, or generated by the second portion C-122 through the spacer C-13), or may be fixed at the atrial septum by helical mechanism on the second portion C-122.

A part or all of the anchoring apparatus C-12 constitutes the second electrode C-114. In other words, the second electrode C-114 and the anchoring apparatus C-12 are integrally formed. That is, the anchoring apparatus C-12 is used for anchoring the pacemaking apparatus C-11 on the atrial septum of the patient, and also serving as one of the electrodes of the pacemaking apparatus.

A part or all of the anchoring apparatus is used as an electrode of the pacemaking apparatus, and there is no need to set a separate electrode Therefore, the structure of the atrial implanting device is simplified. A complex structure will increase the adsorption of floating objects in the blood and the neoplasm of the tissue, which may relatively increase the occurrence of adverse reactions.

It should be understood that, in other embodiments, the anchoring apparatus may not be used as the second electrode. For example, a second electrode may be provided separately. For another example, a part or all of the housing of the pacemaking apparatus may constitute the second electrode.

In some embodiments, referring to FIG. 41, the atrial implanting device C-10 further includes a spacer C-13. The spacer C-13 is located between the first electrode C-113 and the anchoring apparatus C-12 (that is, the second electrode C-114). The spacer C-13 may be made of an insulating material, to electrically separate the first electrode C-113 from the anchoring apparatus C-12 (that is, the second electrode C-114).

In this way, direct contact (direct electrical connection) between the two electrodes is avoided.

It should be understood that, the embodiment of the present disclosure does not specifically limit the formation and size of the spacer C-13, as long as the first electrode C-113 and the anchoring apparatus C-12 is electrically separated, to avoid direct contact between the first electrode C-113 and the anchoring apparatus C-12.

In some embodiments, referring to FIG. 41, the anchoring apparatus C-12 has a mesh frame.

Most cardiac implants have a mesh frame. Clinical research shows that the mesh frame is not easy to cause adverse reactions. Therefore, by adopting the anchoring apparatus having the mesh frame, the influence of the atrial implanting device on the physiological function of the atria is reduced, and the possibility of adverse reactions is reduced.

Optionally, in an embodiment in which the anchoring apparatus has a mesh frame, an outer side of the anchoring apparatus may be coated with a coating layer beneficial to endothelialization, to accelerate the formation of endothelialization.

Optionally, in an embodiment in which the anchoring apparatus serves as the second electrode and the coating layer is made of an insulating material, the anchoring apparatus may have a bare portion that is not covered by the coating layer and is directly exposed to the blood in the atria, or the anchoring apparatus may be connected to a conductive member that is conductive and is directly in contact with the blood in the atrium, to achieve the function as the second electrode.

In some embodiments, referring to FIG.41 and FIG.42, the housing C-111 of the pacemaking apparatus C-11 is configured to be disposed in the right atrium of the patient.

The housing of the pacemaking apparatus is configured in the right atrium of the patient, so that a space occupied by the atrial implanting device in the left atrium is reduced. Due to the higher pressure of the left atrial, the portion of the atrial implanting device placed in the left atrium is more easily to cause turbulence, thereby inducing thrombosis. Therefore, by reducing the space occupied by the atrial implanting device in the left atrium, the influence on the blood flow in the left atrium is reduced, and thus the risk of thrombosis is reduced.

It should be noted that, in other embodiments, the housing of the pacemaking apparatus may also be configured in the left atrium of the patient.

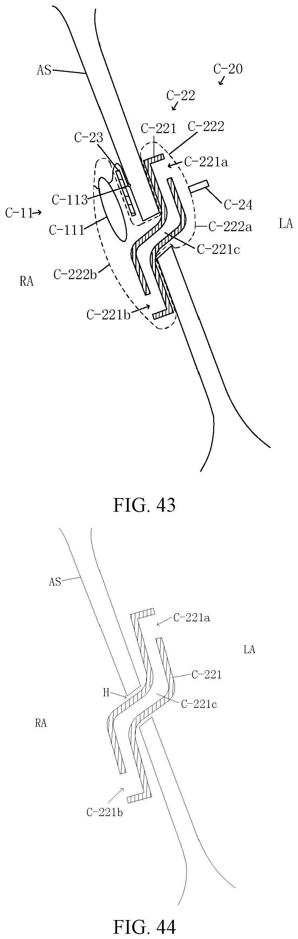

FIG. 43 is a schematic diagram of an atrial implanting device C-20 according to another embodiment of the present disclosure.

The atrial implanting device C-20 and the atrial implanting device C-10 have many same elements, and for the sake of brevity, the same elements are marked with the same reference numerals, and the corresponding descriptions are omitted.

Referring to FIG. 43, the atrial implanting device C-20 includes a shunt apparatus C-22. The shunt apparatus C-22 is configured at an opening of the atrial septum of the patient. The shunt apparatus C-22 is used for communicating with the left atrium and the right atrium through the opening at the atrial septum.

The housing C-111 of the pacemaking apparatus is mounted on the shunt apparatus C-22, thereby being anchored to the atrial septum of the patient. That is, the anchoring apparatus of the atrial implanting device C-20 is the shunt apparatus C-22. The shunt apparatus C-22 is used for communicating with the left atrium and the right atrium of the patient through the opening at the atrial septum of the patient, and is also used for anchoring the housing C-111 of the pacemaking apparatus to the atrial septum of the patient.

For a heart failure patient with a continuous high pressure of the left atrium, an opening may be formed at the atrial septum through ostomy, and an atrial shunt apparatus is mounted at the opening, so that the left atrium and the right atrium of the patient communicate with each other, to make the blood in the left atrium is shunted to the right atrium, and thus the pressure of the left atrium is reduced, and the symptom of the heart failure is alleviated.

In some cases, such patients also need to perform pacing therapy. If a traditional pacemaking apparatus is used, the patient needs to purchase and implant the pacemaking apparatus separately, which will increase the cost of treatment. In addition, the traditional pacemaking apparatuses need to be anchored to the heart tissue of the patient by a separate anchoring apparatus, which will cause additional damage to the heart tissue.

The atrial implanting device provided in the present embodiment of the present disclosure has a shunting function and a pacing function, and the patient does not need to purchase and implant the shunt apparatus and the pacemaking apparatus separately, so that the treatment cost of the patient is reduced. In addition, in the embodiment of the present disclosure, the shunt apparatus is used as an anchoring apparatus, which does not form additional damage on the heart tissue of the patient caused by anchoring the pacemaking apparatus, thereby facilitating the health of the patient.

In some embodiments, referring to FIG. 43, the shunt apparatus C-22 includes a shunt member C-221. The shunt member C-221 forms a first opening C-221a, a second opening C-221b and a shunt pathway C-221c extending between the two (that is, between the first opening C-221a and the second opening C-221b). The first opening C-221a is configured to be located in the left atrium of the patient, and the second opening C-221b is configured to be located in the right atrium of the patient, and thus the left atrium communicates with the right atrium of the patient.

In some embodiments, referring to FIG.43, two electrodes of the pacemaking apparatus C-11 may be integrally formed with the shunt member C-221. In other words, a part or all of the shunt member C-221 constitutes the second electrode of the pacemaking apparatus C-11. That is, the shunt member C-221 is used for implementing a shunting function, and serving as an electrode of the pacemaking apparatus C-11.

A part or all of the shunt member is used as an electrode of the pacemaking apparatus, and one electrode does not need to be separately provided. Therefore, the structure of the atrial implanting device is simplified. A complex structure will increase the adsorption of floating objects in the blood and the neoplasm of the tissue, which may cause adverse reactions.

In some embodiments, referring to FIG. 43, the shunt apparatus C-22 may further include a mesh frame C-222. The mesh frame C-222 covers the housing C-111 of the shunt member C-221 and the pacemaking apparatus C-11.

The mesh frame C-222 may be integrally formed with the shunt member C-221, and the mesh frame C-222 and the shunt member C-221 may also be two separate members, which is not specifically limited in the embodiment of the disclosure.

By arranging the mesh frame, the position and posture of the shunt member may be better fixed. In addition, most of the penetrating atrial shunt apparatuses adopt a mesh frame. Clinical research indicates that this mesh frame is not easy to cause an adverse reaction. Therefore, by covering the housing of the shunt member and the pacemaking apparatus in the mesh frame, the influence of the shunt part on the physiological function of the atria is reduced, and thus the possibility of the adverse reaction is reduced.

It should be noted that, in some embodiments, the shunt apparatus may not include a mesh frame. It should also be noted that, in some embodiments, the shunt member may also extend out of the mesh frame, so that the first opening and the second opening are located outside the mesh frame.

In some embodiments, referring to FIG. 43, the two electrodes of the pacemaking apparatus C-11 may be integrally formed with the mesh frame C-222. In other words, a part or all of the mesh frame C-222 constitutes the second electrode of the pacemaking apparatus C-11.

A part or all of the mesh frame is used as an electrode of the pacemaking apparatus, and an electrode does not need to be separately arranged. Thus, the structure of the atrial implanting device is simplified. A complex structure will increase the adsorption of floating objects in the blood and the neoplasm of the tissue, which may cause the adverse reaction.

In some embodiments, referring to FIG.43, the atrial implanting device C-22 further includes a spacer C-23. The spacer C-23 is located between the first electrode C-113 and the mesh frame C-222 (that is, the second electrode), and thereby the first electrode C-113 and the mesh frame C-222 (that is, the second electrode) are electrically separated.

In some embodiments, referring to FIG. 43, the spacer C-23 is located between the first electrode C-113 and the shunt member of the right atrium or the left atrium, and thereby the first electrode C-113 is electrically separated from other structures of the implanting member.

In this way, direct contact of the two electrodes is avoided.

In some embodiments, referring to FIG. 43, the housing C-111 of the pacemaking apparatus C-11 is configured in the right atrium of the patient.

Since the mesh frame covers the housing of the pacemaking apparatus, by configuring the housing of the pacemaking apparatus in the right atrium of the patient, a space occupied by the portion of the mesh frame located in the left atrium is reduced. Since the implantation in the left atrium is more prone to induce turbulence, and thus the thrombus is formed. Therefore, in this manner, the risk of formation of the thrombus is reduced.

In some embodiments, referring to FIG. 43, the atrial implanting device C-20 further includes a collection apparatus C-24. The collection apparatus C-24 is used for collecting activity information of the heart of the patient. For example, the activity information of the heart may, but is not limited to, one or more of blood pressure information, blood flow rate information, blood pH information, blood temperature information, blood oxygen information, heart sound information, and cardiac contractility information in the heart of the patient.

The collection apparatus C-24 is mounted at the shunt apparatus C-22 (that is, the anchoring apparatus), so as to be anchored to the atrial septum of the patient. It should be understood that, although in FIG.43, the collection apparatus C-24 is located in the left atrium, in other embodiments, the collection apparatus C-24 may also be located in the right atrium.

It should also be understood that, although in FIG. 43, the collection apparatus C-24 is mounted on the mesh frame C-222, in other embodiments, the collection apparatus C-24 may also be mounted on other positions. For example, in some embodiments, the collection apparatus C-24 may be mounted on the shunt member C-221. For another example, in some embodiments, the collection apparatus C-24 may also be mounted on the housing C-111 of the pacemaking apparatus C-11.

It should be understood that, in the embodiment shown in FIG. 41, the atrial implanting device C-10 may also include a collection apparatus, and for example, the collection apparatus may be mounted on the anchoring apparatus C-12 or the housing C-111 of the pacemaking apparatus C-11.

The collection apparatus collects the activity information of the atria of the patient, to monitor the health condition of the patient, such as hemodynamic information. By using the same anchoring apparatus to anchor the pacemaking apparatus and the collection apparatus, the anchoring apparatuses of the pacemaking apparatus and the collection apparatus does not need to be set respectively, and thus the additional damage to the cardiac tissue caused by a plurality of anchoring apparatuses is avoided.

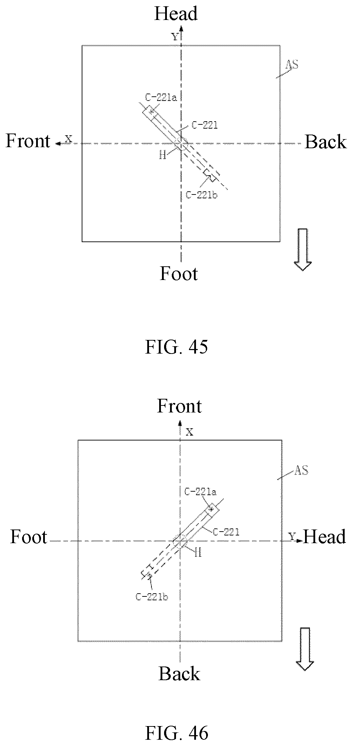

FIG. 44 is a schematic diagram of a shunt member C-221according to an embodiment of the present disclosure. FIG. 45 and FIG. 46 are schematic diagrams of the shunt member C-221 in FIG. 44 from other view directions. The view direction of FIG. 45 is a view direction viewed from a left side to a right side of a patient when the patient is in an upright posture. The view direction of FIG. 46 is a view direction viewed from the left side to the right side of the patient when the patient is in a supine posture. In FIG. 45 and FIG. 46, the solid portion of the shunt member C-221 represents a portion arranged in the left atrium of the patient, and the dashed line portion represents a portion arranged in the right atrium of the patient.

In some embodiments, referring to FIGs. 44 to 46, when the shunt member C-221 is mounted in the atria of the patient, a first opening C-221a may be configured to be closer to the front and the head of the body of the patient relative to a second opening C-221b.

If the first opening C-221a is closer to the head and the front of the body of the patient relative to the second opening C-221b, then the first opening C-221a is higher than the second opening C-221b in a direction of gravity when the patient is in an upright posture and a supine posture (or a posture close to the upright posture or the supine posture), and thus the blood and/or the suspended substance (such as micro-thrombus) in the right atrium needs to overcome the gravity to reach the left atrium.

Consequently, when the patient is in the upright posture or the supine posture (or a posture close to the upright posture or the supine posture), the shunt member C -221 provided by this embodiment effectively prevents or reduces the blood and/or the suspended substance in the blood from entering into the left atrium from the right atrium. Compared with setting a valve, this method avoids the problem of blockage of the pathway. In daily life, a person is in the upright posture or the supine posture (or a posture close to the upright posture or the supine posture) at most of the time. Therefore, the shunt member C-221 provided by this embodiment meets the needs of daily activities of the patient.

In some embodiments, referring to FIGs. 44 to 46, when the shunt member C-221 is mounted in the atria of the patient, the first opening C-221a may be configured to be closer to the head and the front of the body of the patient relative to the hole H at the atrial septum, and the second opening C-221b may be configured to be closer to the foot and the back of the body of the patient relative to the hole H at the atrial septum.

In order to more clearly and accurately describe this implementation, a hypothetical plane rectangular coordinate system is constructed, to describe the implementation from another perspective. The coordinate system takes a plane where the atrial septum AS is located as a reference surface, takes a position where the hole H is located as an origin, takes an intersection line of a horizontal plane passing through the hole H when the patient is in the upright posture and the plane where the atrial septum AS is located as an X axis, takes a direction from a back to the front of the body of the patient as a positive direction of the X axis, , takes a straight line passing through the origin and perpendicular to the X axis, on the reference surface, as a Y axis, and takes a direction from a foot to the head of the patient as a positive direction of the Y axis.

It should be understood that, the atrial septum of the patient is not an ideal plane, the atrial septum has a certain thickness and is not absolutely flat. However, in order to clearly describe the embodiments of the present disclosure as much as possible, it may be considered that the atrial septum is located on a certain plane. For example, a plane where the atrial septum is located may refer to a plane with the highest degree of coincidence with the atrial septum. For example, the plane where the atrial septum is located may also be a fitted plane (or a hypothetical plane), and a sum of distances from all points of the atrial septum to the plane (shortest distance) is minimum.

When the shunt member C-221 is mounted in the atria of the patient, the first opening A-221a is configured so that an orthographic projection of the first opening on the plane where the atrial septum is located is located in a first quadrant of this coordinate system, and the second opening A-221b is configured so that an orthographic projection of the second opening on the plane where the atrial septum is located is located in a third quadrant of this coordinate system.

As shown in FIG. 45, when the patient is in an upright posture, the first opening A-221a is higher than the second opening A-221b in a direction of gravity (that is, a direction indicated by a thick arrow in the figure).

As shown in FIG. 46, when the patient is in an upright posture, the first opening A-221a is higher than the second opening A-221b in the direction of the gravity (that is, a direction indicated by a thick arrow in the figure).

It can be seen therefrom that, in this way, the first opening A-111a is closer to the head and the front of the body of the patient relative to the second opening, so that when the patient is in the upright posture or the supine posture (or a posture close to the supine or the upright posture), the blood and/or the suspended substance in the blood is prevented from entering into the left atrium from the right atrium.

It should be understood that, the shunt apparatus in the aforementioned embodiments is merely an exemplary implementation. For an implementation of the shunt apparatus, the present disclosure is not specifically limited, as long as the function of communicating with the left atrium and the right atrium of the patient is achieved. In some embodiments, the shunt apparatus in the embodiment of the present disclosure may also use a conventional shunt apparatus.

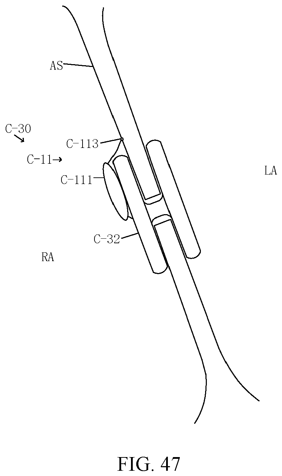

FIG. 47 is a schematic diagram of an atrial implanting device C-30 according to another embodiment of the present disclosure.

The atrial implanting device C-30 and the atrial implanting device C-10 have many same elements, and for the sake of brevity, the same elements are marked with the same reference numerals, and the corresponding descriptions are omitted.

Referring to FIG. 47, the atrial implanting device C-30 includes a plugging apparatus C-32. The plugging apparatus C-32 is configured at an opening of the atrial septum of the patient. The plugging apparatus C-32 is configured to plug the opening at the atrial septum of the patient, so that the left atrium and the right atrium of the patient do not communicate with each other.

The housing C-111 of the pacemaking apparatus C-11 is mounted on the plugging apparatus C-32, so that the housing C-111 of the pacemaking apparatus C-11 is anchored to the atrial septum of the patient. That is, the anchoring apparatus of the atrial implanting device C-30 is the plugging apparatus C-32. The plugging apparatus C-32 is used for plugging the opening at the atrial septum of the patient, and is also used for anchoring the housing C-111 of the pacemaking apparatus C-11 to the atrial septum of the patient.

It should be understood that, although in FIG. 47, the housing C-111 of the pacemaking apparatus C-11 is configured in the right atrium of the patient, in other embodiments, the housing C-111 of the pacemaking apparatus C-11may also be configured in the left atrium of the patient.

It should also be understood that, in other embodiments, the atrial implanting device C-30 may further include a collection apparatus, and the collection apparatus may be mounted on the plugging apparatus C-32, or may be mounted at the housing C-111 of the pacemaking apparatus C-11.

For a patient with an atrial septum defect, a plugging apparatus may be disposed at the opening of the atrial septum, to plug blood circulation between the left atrium and the right atrium, and thus a normal blood circulation path is restored.

Under some cases, such patients also need to perform pacing therapy. If a conventional pacemaking apparatus is used, the patient needs to purchase and implant the pacemaking apparatus separately, which will increase the cost of treatment. In addition, the conventional pacemaking apparatus need to be anchored to the heart tissue of the patient by a separate anchoring apparatus, which may cause additional damage to the heart tissue.

The atrial implanting device provided in the embodiment of the present disclosure has both a plugging function and a pacing function, and the patient does not need to purchase and implant the plugging apparatus and the pacemaking apparatus separately, so that the cost of the treatment of the patient is reduced. In addition, in the embodiment of the present disclosure, the plugging apparatus is used as an anchoring apparatus, and additional damage of the heart tissue of the patient caused by anchoring the pacemaking apparatus is avoided, thereby facilitating the health of the patient.

It should be understood that, the plugging apparatus in the aforementioned embodiments is merely an exemplary implementation. For an implementation of the plugging apparatus, the present disclosure is not specifically limited, as long as the function of communicating with the left atrium and the right atrium of the patient is achieved. In some embodiments, the plugging apparatus in the embodiment of the present disclosure may also use a conventional plugging apparatus.

It should be understood that, the term "include" and its variants used in the present disclosure are open-ended inclusion, that is, "include but not limited to". The term "according to" is "at least partially according to". The term "an embodiment" means "at least one embodiment"; and the term "another embodiment" means "at least one other embodiment".

It should be understood that, although the terms "first" or "second" and the like may be used in the present disclosure to describe various elements (such as a first xx and a second xx), these elements are not intended to limit by these terms, and these terms are merely used to distinguish one element from another element.

It should be understood that, the term "include" and its variants used in the present disclosure are open-ended inclusion, that is, "include but not limited to". The term "according to" is "at least partially according to". The term "an embodiment" means "at least one embodiment"; and the term "another embodiment" means "at least one other embodiment".

The foregoing descriptions are merely specific implementations of the present disclosure, but the protection scope of the present disclosure is not limited thereto. Any changes or substitutions may be easily conceived of by a person skilled in the art within the technical scope disclosed in the present disclosure, will be covered by the protection scope of the present disclosure. Therefore, the protection scope of the present disclosure shall be subject to the protection scope of the claims.

## Claims

1. An atrial shunt apparatus, which is used for shunting blood to a right atrium from a left atrium through a hole at an atrial septum of a patient, wherein the atrial shunt apparatus comprises a shunt part, the shunt part defines a shunt pathway, and the shunt pathway has a first opening and a second opening; and
the shunt pathway is configured to communicate with the left atrium and the right atrium through the first opening and the second opening respectively, the first opening is configured to be closer to a head of the patient relative to the second opening, and/or the first opening is configured to be closer to a front of a body of the patient relative to the second opening.

2. The atrial shunt apparatus according to claim 1, wherein the first opening is configured to be closer to the head and the front of the body of the patient relative to the second opening.

3. The atrial shunt apparatus according to claim 2, wherein the first opening is configured to be closer to the head and the front of the body of the patient relative to the hole, and the second opening is configured to be closer to a foot and a back of the body of the patient relative to the hole.

4. The atrial shunt apparatus according to claim 2, wherein the first opening is configured so that an orthographic projection of the first opening on a plane where the atrial septum is located is located in a first quadrant of a hypothetical coordinate system, and the second opening is configured so that an orthographic projection of the second opening on the plane where the atrial septum is located is located in a third quadrant of the coordinate system; and
the coordinate system is a plane rectangular coordinate system, the coordinate system takes the plane where the atrial septum is located as a reference surface, takes the hole as an origin, takes an intersection line of a horizontal plane passing through the origin when the patient is in an upright posture and the reference surface as an X axis, takes a direction from a back to the front of the body of the patient as a positive direction of the X axis, takes a straight line passing through the origin and perpendicular to the X axis, on the reference plane, as a Y axis, and takes a direction from a foot to the head of the body of the patient as a positive direction of the Y axis.

5. The atrial shunt apparatus according to claim 4, wherein the first opening is configured so that an included angle, between the X axis and a connecting line between an orthographic projection of the first opening on the reference surface and the origin, ranges from 30 degrees to 60 degrees, and the second opening is configured so that an included angle, between the X axis and a connecting line between an orthographic projection of the second opening on the reference surface and the origin, ranges from 30 degrees to 60 degrees.

6. The atrial shunt apparatus according to claim 5, wherein the first opening is configured so that the included angle, between the X axis and the connecting line between the orthographic projection of the first opening on the reference surface and the origin, is 45 degrees, and the second opening is configured so that the included angle, between the X axis and the connecting line between the orthographic projection of the second opening on the reference surface and the origin, is 45 degrees.

7. The atrial shunt apparatus according to claim 2, wherein the first opening is configured so that, an orthographic projection, of the first opening on a plane where the atrial septum is located, at least partially coincides with the hole, and the second opening is configured to be closer to a foot and a back of the body of the patient relative to the hole.

8. The atrial shunt apparatus according to claim 2, wherein the first opening is configured so that, an orthographic projection of the first opening on a plane where the atrial septum is located, substantially coincides with an origin of a hypothetical coordinate system, and the second opening is configured so that an orthographic projection of the second opening on the plane where the atrial septum is located, is located in a third quadrant of the coordinate system; and
the coordinate system is a plane rectangular coordinate system, the coordinate system takes the plane where the atrial septum is located as a reference plane, takes the hole as an origin, takes an intersection line of a horizontal plane passing through the origin when the patient is in an upright posture and the reference plane as an X axis, takes a direction from a back to the front of the body of the patient as a positive direction of the X axis, takes a straight line passing through the origin and perpendicular to the X axis, on the reference surface, as a Y axis, and takes a direction from a foot to the head of the body of the patient as a positive direction of the Y axis.

9. The atrial shunt apparatus according to claim 8, wherein the second opening is configured so that, an included angle, between the X axis and a connecting line between an orthographic projection of the second opening on the reference surface and the origin, ranges from 30 degrees to 60 degrees.

10. The atrial shunt apparatus according to claim 9, wherein the second opening is configured so that, the included angle, between the X axis and the connecting line between the orthographic projection of the second opening on the reference surface and the origin, is 45 degrees.

11. The atrial shunt apparatus according to claim 1, wherein the shunt part is tubular, the shunt part comprises a first portion pipe body and a second portion pipe body, the first portion pipe body is configured to be arranged in the left atrium, the second portion pipe body is configured to be arranged in the right atrium, the first opening is arranged on an end face or a peripheral wall of the first portion pipe body, and the second opening is arranged on an end face or a peripheral wall of the second portion pipe body.

12. The atrial shunt apparatus according to claim 1, wherein the shunt part is tubular, the shunt part comprises a first portion pipe body and a second portion pipe body, the first portion pipe body is configured to be arranged in the left atrium, the second portion pipe body is configured to be arranged in the right atrium, the first opening and the second opening are respectively arranged on the first portion pipe body and the second portion pipe body, and volume of the first portion pipe body is less than volume of the second portion pipe body.

13. The atrial shunt apparatus according to claim 1, wherein the shunt part is tubular, the shunt part comprises a first portion pipe body and a second portion pipe body, the first portion pipe body is configured to be arranged in the left atrium, the second portion pipe body is configured to be arranged in the right atrium, the first opening and the second opening are respectively arranged on the first portion pipe body and the second portion pipe body, and the first portion pipe body is shorter than the second portion pipe body.

14. The atrial shunt apparatus according to claim 2, wherein the first opening is configured to be higher than the second opening in a direction of gravity when the patient is in an upright and a supine posture, and a height difference between the first opening and the second opening in the direction of the gravity ranges from 1.5 cm to 2 cm.

15. The atrial shunt apparatus according to any one of claims 1 to 14, wherein an inner diameter of the shunt pathway ranges from 8 mm to 15 mm.

16. The atrial shunt apparatus according to any one of claims 1 to 14, wherein the inner wall of the shunt pathway is hydrophobic and/or resistant to formation of neoplasma.

17. The atrial shunt apparatus according to claim 1, wherein the atrial shunt device further comprises a frame part, the frame part has a mesh frame, and the frame part comprises:
a first frame part, which is configured to be arranged in the left atrium;
a second frame part, which is configured to be arranged in the right atrium; and
a connecting part, which connects the first frame part and the second frame part, and is configured to pass through the hole, wherein the shunt part is tubular, and the shunt part comprises:
a first portion pipe body, the first opening is arranged on the first portion pipe body, and the first portion pipe body is configured to be surrounded by the first frame part; and
a second portion pipe body, the second opening is arranged on the second portion pipe body, and the second portion pipe body is configured to be surrounded by the second frame part.

18. The atrial shunt apparatus according to claim 1, wherein the atrial shunt apparatus further comprises a frame part, the frame part comprises:
a first frame part, which is configured to be arranged in the left atrium;
a second frame part, which is configured to be arranged in the right atrium; and
a connecting part, which connects the first frame part and the second frame part, and is configured to pass through the hole, wherein the shunt part is tubular, and the shunt part comprises:
a first portion pipe body, the first opening is arranged on the first portion pipe body, the first portion pipe body is configured so that a portion of the first portion pipe body is arrange in the first frame part, and the other portion of the first portion pipe body extends out of the first frame part, to make the first opening be exposed out of the frame part; and
a second portion pipe body, the second opening is arranged on the second portion pipe body, the second portion pipe body is configured so that a portion of the second portion pipe body is arrange in the second frame part, and the other portion of the second portion pipe body extends out of the second frame part, to make the second opening be exposed to the outside of the frame part.

19. The atrial shunt apparatus according to claim 1, wherein the atrial shunt apparatus further comprises a frame part, and the frame part comprises:
a first frame part, which is configured to be arranged in the left atrium;
a second frame part, which is configured to be arranged in the right atrium; and
a connecting part, which connects the first frame part and the second frame part, and is configured to pass through the hole, wherein the shunt part is tubular, and the shunt part comprises:
a first portion pipe body, which is configured to extend from an inner of the first frame part to a surface of the first frame part, and form the first opening on the surface of the first frame part; and
a second portion pipe body, which is configured to extend from an inner of the second frame part to a surface of the second frame part, and form the second opening on the surface of the second frame part.

20. The atrial shunt apparatus according to any one of claims 17 to 19, wherein the first frame part is configured so that, an orthographic projection, of the first frame part on a plane where the atrial septum is located, covers an orthographic projection, of the first portion pipe body and the second portion pipe body on the plane where the atrial septum is located, and the second frame part is configured so that, an orthographic projection, of the second frame part on the plane where the atrial septum is located, covers the orthographic projection of the first portion pipe body and the second portion pipe body on the plane where the atrial septum is located.

21. The atrial shunt apparatus according to any one of claims 17 to 19, wherein the first portion pipe body is configured so that, an orthographic projection, of the first portion pipe body on a plane where the atrial septum is located, is at least partially located outside an orthographic projection of the second frame part on the plane where the atrial septum is located, and an orthographic projection, of the second portion pipe body on the plane where the atrial septum is located, is at least partially located outside an orthographic projection of the first frame part on the plane where the atrial septum is located.

22. The atrial shunt apparatus according to any one of claims 17 to 19, wherein the first frame part is less than the second frame part.

23. The atrial shunt apparatus according to any one of claims 17 to 19, wherein the first frame part and/or the second frame part is dome-shaped.

24. An atrial shunt apparatus, which is used for shunting blood to a right atrium from a left atrium through a hole at an atrial septum of a patient, wherein the atrial shunt apparatus comprises a shunt part, the shunt part defines a shunt pathway, the shunt pathway has a first opening and a second opening; and
the shunt pathway is configured to communicate with the left atrium and the right atrium through the first opening and the second opening respectively, the first opening is configured to be higher than the second opening in a direction of gravity when the patient is in an upright posture, and/or the first opening is configured to be higher than the second opening in a direction of gravity when the patient is in a supine posture.

25. An atrial shunt apparatus, wherein the atrial shunt apparatus comprises a shunt part, the shunt part defines a shunt pathway, the shunt pathway has a first opening and a second opening, the shunt part is tubular, wherein the shunt part comprises:
a first portion pipe body, the first opening is arranged on the first portion pipe body;
a second portion pipe body, the second opening is arranged on the second portion pipe body; and
a third portion pipe body, an end of the third portion pipe body communicates with the first portion pipe body, the other end of the third portion pipe body communicates with the second portion pipe body, the first portion pipe body is substantially parallel to the second portion pipe body, and the first portion pipe body and the second portion pipe body form an included angle with the third portion pipe body respectively; and the first portion pipe body and the second portion pipe body respectively extend from the third portion pipe body along opposite directions.

26. An atrial shunt apparatus, wherein the atrial shunt apparatus comprises a shunt part, the shunt part defines a shunt pathway, the shunt pathway has a first opening and a second opening, wherein the shunt part is tubular, and the shunt part comprises:
a first portion pipe body, the first opening is arranged on an end face of the first portion pipe body; and
a second portion pipe body, the second opening is arranged on the second portion pipe body, an included angle is formed between the second portion pipe body and the first portion pipe body, and the first portion pipe body is shorter than the second portion pipe body.

27. A method for mounting an atrial shunt apparatus, wherein the method for mounting the atrial shunt apparatus comprises:
providing the atrial shunt apparatus, wherein the atrial shunt apparatus is used for shunting blood from a left atrium to a right atrium through a hole at an atrial septum of a patient, the atrial shunt apparatus comprises a shunt part, the shunt part defines a shunt pathway, and the shunt pathway has a first opening and a second opening; and
mounting the atrial shunt apparatus to atria of the patient, wherein the shunt pathway is configured to communicate with the left atrium and the right atrium through the first opening and the second opening respectively, the first opening is configured to be closer to a head of the patient relative to the second opening, and/or the first opening is configured to be closer to a front of a body of the patient relative to the second opening.

28. A method for mounting an atrial shunt apparatus, wherein, the method for mounting the atrial shunt apparatus comprises:
providing the atrial shunt apparatus, wherein the atrial shunt apparatus is used for shunting blood from a left atrium to a right atrium through a hole at an atrial septum of a patient, the atrial shunt apparatus comprises a shunt part, the shunt part defines a shunt pathway, and the shunt pathway has a first opening and a second opening; and
mounting the atrial shunt apparatus in atria of the patient, wherein the shunt pathway is configured to communicate with the left atrium and the right atrium through the first opening and the second opening respectively, the first opening is configured to be higher than the second opening in a direction of gravity when the patient is in an upright and a supine posture, and/or the first opening is configured to be higher than the second opening in a direction of gravity when the patient is in a supine posture.

29. An atrial implanting device, wherein the atrial implanting device comprises:
a treatment apparatus, which is configured to be arranged at an opening of an atrial septum of a patient, and is used for allowing blood to flow between a left atrium and a right atrium of the patient, or preventing blood from flowing between a left atrium and a right atrium of the patient; and
a collection apparatus, which is configured to be arranged on the treatment apparatus, and is used for collecting activity information of a heart of the patient.

30. The atrial implanting device according to claim 29, wherein the collection apparatus comprises a hemodynamic information collection unit, and the activity information comprises hemodynamic information collected by the hemodynamic information collection unit.

31. The atrial implanting device according to claim 30, wherein the hemodynamic information comprises pressure information, the hemodynamic information collection unit comprises a vibrating diaphragm for sensing the pressure information, and the vibrating diaphragm is configured to be directly exposed to blood in the left atrium or be directly exposed to blood in the right atrium.

32. The atrial implanting device according to claim 31, wherein the vibrating diaphragm is configured to be parallel to the atrial septum.

33. The atrial implanting device according to claim 31, wherein the hemodynamic information collection unit has a first end and a second end which are opposite to each other, the vibrating diaphragm is arranged at the first end, and a direction from the first end to the second end of the hemodynamic information collection unit is configured to be the same as or opposite to a blood flow direction.

34. The atrial implanting device according to claim 31, wherein the hemodynamic information collection unit has a first end and a second end which are opposite to each other, the vibrating diaphragm is arranged at the first end, a direction from the first end to the second end is perpendicular to the atrial septum, and the first end is farther from the atrial septum relative to the second end.

35. The atrial implanting device according to claim 31, wherein the treatment apparatus comprises a shunt member, the shunt member is provided with a first opening, a second opening and a shunt pathway which extends from the first opening to the second opening, the first opening and the second opening are configured to be respectively located in the left atrium and the right atrium, to allow the blood to flow between the left atrium and the right atrium, and the hemodynamic information collection unit is configured to be located at an edge of the first opening or the second opening.

36. The atrial implanting device according to claim 31, wherein the treatment apparatus comprises a shunt member, the shunt member defines a shunt pathway for communicating with the left atrium and the right atrium, to allow the blood to flow between the left atrium and the right atrium, and the hemodynamic information collection unit is configured to be located in the shunt pathway.

37. The atrial implanting device according to claim 36, wherein the collection apparatus comprises two hemodynamic information collection units, the two hemodynamic information collection units are configured to be located in the shunt pathway, and in the shunt pathway, in a direction from the left atrium to the right atrium, a vibrating diaphragm of one hemodynamic information collection unit is located at an upstream side of the one hemodynamic information collection unit, and a vibrating diaphragm of the other one hemodynamic information collection unit is located at a downstream side of the other one hemodynamic information collection unit.

38. The atrial implanting device according to claim 37, wherein a housing of the two hemodynamic information collection units is integrally formed.

39. The atrial implanting device according to claim 30, wherein the collection apparatus further comprises an intracardiac electrocardiogram collection unit, and the activity information further comprises intracardiac electrocardiogram information collected by the intracardiac electrocardiogram collection unit.

40. The atrial implanting device according to claim 39, wherein the intracardiac electrocardiogram collection unit comprises a first electrode, and a part or all of a housing of the hemodynamic information collection unit constitutes the first electrode.

41. The atrial implanting device according to claim 40, wherein the intracardiac electrocardiogram collection unit further comprises a second electrode, the housing of the hemodynamic information collection unit comprises a first portion housing and a second portion housing which are electrically separated from each other, the first portion housing constitutes the first electrode, and the second portion housing constitutes the second electrode.

42. The atrial implanting device according to claim 39, wherein the treatment apparatus comprises a shunt member, the shunt member defines a shunt pathway for connecting the left atrium and the right atrium, to allow blood to flow between the left atrium and the right atrium, the intracardiac electrocardiogram collection unit comprises a first electrode, and a part or all of the shunt member constitutes the first electrode.

43. The atrial implanting device according to claim 42, wherein the intracardiac electrocardiogram collection unit further comprises a second electrode, the second electrode is configured to be in direct contact with the atrial septum, the atrial implanting apparatus further comprises a spacer, and the spacer is configured to electrically separate the second electrode from the shunt member.

44. The atrial implanting device according to claim 39, wherein the treatment apparatus comprises a shunt member and a frame, the shunt member defines a shunt pathway for communicating with the left atrium and the right atrium, to allow blood to flow between the left atrium and the right atrium, the frame is used for supporting the shunt member, the intracardiac electrocardiogram collection unit comprises a first electrode, and a part or all of the frame constitutes the first electrode.

45. The atrial implanting device according to claim 44, wherein the intracardiac electrocardiogram collection unit further comprises a second electrode, the frame comprises a first frame part and a second frame part which are electrically separated from each other, the first frame part and the second frame part are configured to be respectively located in the left atrium and the right atrium, the first frame part constitutes the first electrode, and the second frame part constitutes the second electrode.

46. The atrial implanting device according to claim 39, wherein the collection apparatus further comprises a calibration unit, and the calibration unit is used for synchronously writing time calibration information into the hemodynamic information and the intracardiac electrocardiogram information respectively.

47. A medical system, wherein the medical system comprises:
the atrial implanting device according to any one of claims 29 to 46; and
an external device which is configured to be located outside a patient, the external device comprises a communication unit, and the communication unit is used for receiving the activity information from the atrial implanting device.

48. The medical system according to claim 47, wherein the external device further comprises a body surface electrocardiogram collection unit, and the body surface electrocardiogram collection unit is used for collecting electrocardiogram information of a body surface of the patient.

49. A medical system, wherein the medical system comprises:
the atrial implanting device according to any one of claims 30 to 38; and
an external device, which is configured to be located outside a patient and comprises a body surface electrocardiogram collection unit and a calibration unit,
wherein the body surface electrocardiogram collection unit is used for collecting body surface electrocardiogram information of the patient; and
the calibration unit is used for generating and sending time calibration information to the hemodynamic information collection unit and the body surface electrocardiogram collection unit, to make the hemodynamic information collection unit and the body surface electrocardiogram collection unit synchronously write the time calibration information into the hemodynamic information and the body surface electrocardiogram information.

50. A medical system, wherein the medical system comprises:
the atrial implanting device according to any one of claims 39 to 45;
an external device, which is configured to be located outside a patient and comprises a body surface electrocardiogram collection unit and a calibration unit,
wherein the body surface electrocardiogram collection unit is used for collecting body surface electrocardiogram information of the patient; and
the calibration unit is used for generating and sending time calibration information to the hemodynamic information collection unit, the intracardiac electrocardiogram collection unit and the body surface electrocardiogram collection unit, to make the hemodynamic information collection unit, the intracardiac electrocardiogram collection unit and the body surface electrocardiogram collection unit synchronously write the time calibration information into the hemodynamic information, the intracardiac electrocardiogram information and the body surface electrocardiogram information.

51. A method for collecting activity information of a heart, comprising:
acquiring hemodynamic information in atria of a patient;
acquiring electrocardiogram information of the patient; and
analyzing the hemodynamic information and the electrocardiogram information, to obtain an analysis result.

52. A medical device, wherein the medical device comprises:
a memory; and
a processor, which is coupled to the memory, wherein the processor is configured to execute the method for collecting the activity information of the heart according to claim 51 based on an instruction stored in the memory.

53. An atrial implanting device, wherein the atrial implanting device comprises:
a pacemaking apparatus, wherein the pacemaking apparatus comprises a pulse generation module, a first electrode and a housing, the pulse generation module is accommodated in the housing, the first electrode is configured to be in contact with an atrial septum, and the pulse generation module is configured to deliver a pacemaking pulse to the atrial septum through the first electrode; and
an anchoring apparatus, which is configured to anchor the housing to the atrial septum.

54. The atrial implanting device according to claim 53, wherein a part or all of the housing constitutes a second electrode of the pacemaking apparatus.

55. The atrial implanting device according to claim 53, wherein the anchoring apparatus comprises a shunt apparatus, the shunt apparatus is configured to communicate with the left atrium and the right atrium through an opening at the atrial septum, and the pacemaking apparatus is arranged on the shunt apparatus.

56. The atrial implanting device according to claim 55, wherein the shunt apparatus comprises a shunt member, the shunt member forms a shunt pathway for communicating with a left atrium and a right atrium, the pacemaking apparatus further comprises a second electrode, and all or a part of the shunt member constitutes the second electrode.

57. The atrial implanting device according to claim 55, wherein the shunt apparatus comprises a shunt member and a mesh frame, the shunt member forms a shunt pathway for communicating with a left atrium and a right atrium, and the mesh frame covers the housing and the shunt member.

58. The atrial implanting device according to claim 57, wherein the pacemaking apparatus further comprises a second electrode, and a part or all of the mesh frame constitutes the second electrode.

59. The atrial implanting device according to claim 58, wherein the atrial implanting device further comprises a spacer, and the spacer is configured between the first electrode and the mesh frame, to electrically separate the first electrode from the mesh frame.

60. The atrial implanting device of claim 55, wherein the shunt apparatus comprises a shunt member, the shunt member is formed with a first opening, a second opening and a shunt pathway which extends between the first opening and the second opening, the first opening is configured in a left atrium of a patient, the second opening is configured in a right atrium of the patient, and the first opening is configured to be closer to a head of the patient and a front of the body of the patient relative to the second opening.

61. The atrial implanting device according to claim 53, wherein the anchoring apparatus comprises a plugging apparatus, the plugging apparatus is configured at an opening of the atrial septum and plugs the opening, and the housing is mounted on the plugging apparatus.

62. The atrial implanting device according to any one of claims 53 to 61, wherein the atrial implanting device further comprises a collection apparatus, the collection apparatus is used for collecting activity information of a heart of the patient, and the anchoring apparatus is configured to anchor the collection apparatus to the atrial septum.

63. The atrial implanting device according to any one of claims 53 to c61, wherein the housing is configured in the right atrium.
